(19) Europäisches Patentamt
European Patent Office
Office européen des brevets

(11) EP 3 148 426 B1

(12) EUROPEAN PATENT SPECIFICATION

(45) Date of publication and mention
of the grant of the patent:
**17.05.2023 Bulletin 2023/20**

(21) Application number: **15799200.9**

(22) Date of filing: **28.05.2015**

(51) International Patent Classification (IPC):
**A61B 5/08** (2006.01) **A61B 5/03** (2006.01)

(52) Cooperative Patent Classification (CPC):
**G16H 40/63; A61B 5/0205; A61B 5/037;
A61B 5/0806; A61B 5/091;** Y02A 90/10

(86) International application number:
**PCT/US2015/033052**

(87) International publication number:
**WO 2015/184187 (03.12.2015 Gazette 2015/48)**

(54) **METHOD, SYSTEM AND SOFTWARE FOR ASSESSING EXTUBATION FAILURE**

VERFAHREN, SYSTEM UND SOFTWARE ZUR SCHÄTZUNG EINES EXTUBATIONSVERSAGENS

PROCÉDÉ, SYSTÈME ET LOGICIEL POUR L'ÉVALUATION D'UN ÉCHEC D'EXTUBATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.05.2014 US 201462004034 P
15.05.2015 US 201562162476 P**

(43) Date of publication of application:
**05.04.2017 Bulletin 2017/14**

(73) Proprietor: **Children's Hospital Los Angeles
Los Angeles, California 90027 (US)**

(72) Inventors:
• **KHEMANI, Robinder
Los Angeles, CA 90027 (US)**
• **FLINK, Rutger
1017 NN Amsterdam (NL)**
• **NEWTH, Christopher JL
Long Beach, CA 90803 (US)**

(74) Representative: **HGF
HGF Limited
1 City Walk
Leeds LS11 9DX (GB)**

(56) References cited:
WO-A1-98/41146     US-A- 5 678 535
US-A1- 2009 221 926     US-A1- 2012 041 279
US-A1- 2014 142 652

• **PATRICK A. ROSS ET AL: "Pressure-rate product
and phase angle as measures of acute inspiratory
upper airway obstruction in rhesus monkeys",
PEDIATRIC PULMONOLOGY., vol. 45, no. 7, 1
July 2010 (2010-07-01), pages 639-644,
XP055433283, US ISSN: 8755-6863, DOI:
10.1002/ppul.21212**
• **V. KAPLAN ET AL: "Detection of inspiratory flow
limitation during sleep by computer assisted
respiratory inductive plethysmography",
EUROPEAN RESPIRATORY JOURNAL., vol. 15,
no. 3, 1 March 2000 (2000-03-01), page 570,
XP055433278, DK ISSN: 0903-1936, DOI:
10.1034/j.1399-3003.2000.15.24.x**

## Description

This invention was made with government support under Grant Number 1K23HL103785 awarded by the National Institutes of Health. The government has certain rights to the invention.

## FIELD OF INVENTION

The present invention relates generally to a method, and software for monitoring a signal from a living subject to measure a function of the living subject. The present invention specifically relates, for example, to a computer method, and software for accurately calibrating respiratory inductance plethysmography (RIP) signals which when combined with esophageal manometry signals determine inspiratory flow limitation and measure effort of breathing before and after extubation of a patient to provide an automated real-time interpretation for the clinician of whether or not the child/infant has upper airway obstruction, and its severity.

## BACKGROUND

The following description includes information that may be useful in understanding the present invention. It is not an admission that any of the information provided herein is prior art or relevant to the presently claimed invention, or that any publication specifically or implicitly referenced is prior art.

While endotracheal intubation is a lifesaving supportive therapy, adults and children frequently develop complications, including post extubation Upper Airway Obstruction (UAO). Post extubation UAO leads to short and long term morbidity, with increased hospital and intensive care unit length of stay, the need for additional therapeutics, occasionally the need for emergent re-intubation, and increased overall health care cost. While long term complications from endotracheal intubation including subglottic stenosis have become increasingly uncommon, there appears to be no decrease in the incidence of post extubation UAO, which on clinical grounds is estimated to be responsible for 1/3 of all pediatric extubation failures.

While post extubation UAO has been described since the early days of endotracheal intubation, there is limited pediatric data surrounding risk factors. This may be, in part, because the diagnosis of post-extubation UAO is based on subjective clinical assessment. We have previously demonstrated that bedside providers frequently disagree on the presence of post-extubation UAO, and this variability may change the perceived risk factors for post-extubation UAO. This complicates accurate assessment of therapeutic or prevention strategies when there is no clear gold standard to diagnose the outcome. This has likely contributed to the inconclusive data surrounding corticosteroids, for example, to prevent post-extubation UAO in children.

There is a need in the art for non-invasive methods for identifying post extubation UAO in children and adults and subsequent risk of re-intubation and identifying patients at high risk for extubation failure from post-extubation UAO or other causes. Such methods are described herein and in United States Application Nos. 62/004,034 and 62/162,476, and further in documents XP055433283, XP055433278 and WO98/41146A1.

## SUMMARY OF THE INVENTION

The following embodiments and aspects thereof are described and illustrated in conjunction with systems, compositions and methods which are meant to be exemplary and illustrative, not limiting in scope.

Provided herein is a method for evaluating medical conditions relating to effort of breathing in a subject in need thereof. The method includes obtaining one or more biometric signals from the subject relating to the subject's breathing; determining a plurality of trigger points for inspiration, based on the one or more biometric signals; calibrating the biometric signals; determining, from the one or more biometric signals, a relative effort of breathing value; and displaying a report comprising an interpretation of the effort of breathing value, wherein the interpretation is updated as additional data relating to the one or more biometric signals is received. In some embodiments, the medical condition is post-extubation failure which may be due to upper airway obstruction, persistent respiratory failure, neuromuscular weakness or a combination thereof. In some embodiments, the subject is intubated.

In some embodiments, the biometric signals comprise respiratory inductance plethysmography (RIP), esophageal manometry, spirometry or a combination thereof. In some embodiments, the trigger points for inspiration may delayed by any one or more of 0.05ms, 0.1ms, 0.15ms, 0.2ms, 0.3ms or a combination thereof after inspiration by the subject.

In some embodiments, the RIP signal is calibrated while the subject is intubated, prior to extubation, so as to allow for RIP calibration in real-time. In various embodiments, RIP signal is calibrated using a method selecting from the group consisting of calibrating RIP comprises calibration of continuous positive airway pressure, a quantitative diagnostic calibration (QDC) method and use of negative inspiratory force (NIF).

In some embodiments, determining the relative effort of breathing value comprises obtaining pressure rate product (PRP). Tthe PRP may be obtained over a pre-determined amount of time of breathing by the subject. In various embodiments, pre-determined amount of time is any one or more of 10 seconds, 20 seconds, 30 seconds, 40 seconds, 50 seconds, 60 seconds, 90 seconds, 120seconds, 150seconds, 180 seconds, 240 seconds, 300 seconds, 360 seconds, or a combination thereof. In some embodiments, the PRP is obtained by identifying the peak and trough change of the esophageal pressure signals for each breath and multiplying by the respiratory rate.

Also provided herein is a method for determining the likelihood of extubation failure in a subject in need thereof by the methods described herein. In various embodiments, extubation failure may be due to upper airway obstruction, persistent elevation in effort of breathing from persistent respiratory disease or neuromuscular weakness.

Further provided herein is a method for determining the severity of post-extubation UAO in a subject in need thereof. The method includes a first and second sensor; obtaining esophageal pressure ($P_{es}$) signals in the subject using the first sensor; obtaining respiratory inductance plethysmography (RIP) signals in the subject using the second sensor; calibrating the signals obtained from the second sensor; determining the presence of inspiratory flow limitation; obtaining a pressure rate product (PRP) for each breath to determine associated relative severity of inspiratory flow; and averaging the relative severity over the number of breaths taken over a period of time to obtain a relative averaged severity score, wherein an increase in the relative averaged severity score compared to normal subject is indicative of increased severity of post-extubation UAO. In an embodiment, the signals from the second sensor are calibrated prior to extubation. In some embodiments, the period of time is any one or more of 10 seconds, 20 seconds, 30 seconds, 40 seconds, 50 seconds, 60 seconds, 90 seconds, 120seconds, 150seconds, 180 seconds, 240 seconds, 300 seconds, 360 seconds, or a combination thereof of breathing by the subject.

In some embodiments, assessing relative severity comprises assigning a severity score for a given range of PRP values and averaging the severity score by the number of breaths taken by the subject over the period of time. In various embodiments, a severity score of zero is assigned if the PRP is less than 250; one is assigned if the PRP is between 250 and less than 500; two is assigned if the PRP is between 500 and less than 1000; three is assigned if the PRP is between 1000 and less than 1500 and four is assigned if the PRP is > 1500. In some embodiments, the inspiratory flow limitation is determined after extubation.

Also provided herein is a method for identifying patients at higher risk of re-intubation after extubation in a subject in need thereof by the methods described herein, wherein an increase in the relative averaged severity score compared to normal subject is indicative of the subject being at higher risk of re-intubation.

Further described herein is a method for predicting extubation failure in subject in need thereof by the methods described herein, wherein an increase in the relative averaged severity score compared to normal subject is indicative of the subject being at higher risk of extubation failure.

In some embodiments, the esophageal pressure and RIP signals are obtained simultaneously. In some embodiments, the esophageal pressure and RIP signals are obtained sequentially. Oobtaining $P_{es}$ signal comprises performing esophageal manometry. In various embodiments, RIP signals comprise determining movement of abdominal (Abd) wall and rib cage (RC) wall.

Further provided herein is a non-transitory computer-readable storage medium storing one or more programs for evaluating medical conditions relating to effort of breathing in a subject in need thereof, the one or more programs for execution by one or more processors of a computer system, the one or more programs comprising instructions for: obtaining one or more biometric signals from the subject relating to the subject's breathing; determining a plurality of trigger points for inspiration, based on the one or more biometric signals; calibrating the biometric signals; determining, from the one or more biometric signals, a relative effort of breathing value; displaying a report comprising an interpretation of the effort of breathing value, wherein the interpretation is updated as additional data relating to the one or more biometric signals is received. In some embodiments, the medical condition is post-extubation failure, which may be due to upper airway obstruction, persistent respiratory failure, neuromuscular weakness or a combination thereof. In various embodiments, the biometric signals comprise respiratory inductance plethysmography (RIP), esophageal manometry, spirometry or a combination thereof. In some embodiments, the trigger points for inspiration may delayed by any one or more of 0.05ms, 0.1ms, 0.15ms, 0.2ms, 0.3ms or a combination thereof after inspiration by the subject. In some embodiments, the subject is intubated. In various embodiments, the RIP signal is calibrated while the subject is intubated, prior to extubation, so as to allow for RIP calibration in real-time. In various embodiments, the RIP signal is calibrated using a method selecting from the group consisting of calibrating RIP comprises calibration of continuous positive airway pressure, a quantitative diagnostic calibration (QDC) method and use of negative inspiratory force (NIF). In various embodiments, determining the relative effort of breathing value comprises obtaining pressure rate product (PRP), wherein the PRP is obtained over a pre-determined amount of time of breathing by the subject. In exemplary embodiments the pre-determined amount of time is any one or more of 10 seconds, 20 seconds, 30 seconds, 40 seconds, 50 seconds, 60 seconds, 90 seconds, 120seconds, 150seconds, 180 seconds, 240 seconds, 300 seconds, 360 seconds, or a combination thereof. In various embodiments, PRP is obtained by identifying the peak and trough of the esophageal pressure signals for each breath and multiplying by the respiratory rate.

## BRIEF DESCRIPTION OF THE DRAWINGS

The accompanying drawings, which are incorporated into this specification, illustrate one or more exemplary embodiments of the inventions disclosed herein and, together with the detailed description, serve to explain the principles and exemplary implementations of these inventions. One of skill in the art will understand that the drawings are illustrative only, and that what is depicted therein may be adapted based on the text of the specification and the spirit and scope of the teachings herein.

Figure 1 provides an overview of the assessment process, according to an embodiment of the present disclosure.

Figure 2 illustrates the determination of the flow trigger, according to an embodiment of the present disclosure.

Figure 3 illustrates the determination of the Pes trigger, according to an embodiment of the present disclosure.

Figure 4a is a flowchart detailing the calibration process, according to an embodiment of the present disclosure.

Figure 4b is an exemplary display of the selection of the calibration area, according to an embodiment of the present disclosure.

Figure 5 illustrates the application of the severity assessment to the weaning process, according to an embodiment of the present disclosure.

Figure 6 is a flowchart detailing the UAO severity assessment, according to an embodiment of the present disclosure.

Figure 7 depicts, in accordance with an embodiment of the invention, an example of supraglottic UAO from a 10 year old patient status post Tonsillectomy and Adenodectomy, with improvement of flow limitation, lower delta esophageal pressure, increased flow during an Airway (Esmark) Manever.

Figure 8 depicts, in accordance with an embodiment of the invention, an example of subglottic UAO, with severe inspiratory flow limitation (left) with no rise in flow despite continual decrease in esophageal pressure. Significant improvement 20 minutes after racemic epinephrine (right).

Figure 9 depicts, in accordance with an embodiment of the invention, UAO incidence as a function of age for all cause UAO, and likely subglottic UAO. The peak for all cause UAO appears near 2 years of age, and is 18 months for subglottic UAO. Subglottic UAO appears very uncommon after 8 years of age.

Figure 10 depicts, in accordance with an embodiment of the invention, characteristic Konno-Mead plots derived from RIP contrasting diaphragmatic weakness or paralysis from intercostal muscle weakness or paralysis.

Figure 11 depicts, in accordance with an embodiment of the invention, Characteristic Konno-Mead plots derived from RIP demonstrating unilateral diaphragm paralysis, from phrenic nerve injury after cardiac surgery. Notice the figure of 8 appearance to the konno mead plot, visualized as a double bump in the Abdominal band.

Figure 12 depicts, in accordance with an embodiment of the invention, data from a 6 month old child who had a liver transplant and demonstrates progressive diaphragmatic fatigue over the course of a spontaneous breathing trial on continuous positive airway pressure (CPAP). Initially, the phase angle and pressure rate product are normal, but over time the patient becomes more asynchronous (higher phase angle) and has a significantly increased pressure rate product.

Figure 13 depicts, in accordance with an embodiment of the invention, patient with recovering pneumonia, who develops significant thoraco abdominal asynchrony as CPAP is decreased, normalizing when CPAP is increased to 8. This patient has restrictive lung disease and is benefiting from the CPAP to normalize end expiratory lung volumes and breathe with normal respiratory mechanics.

Figure 14 depicts, in accordance with an embodiment of the invention, 3 month old with infantile botulism, who has classic clockwise konno mead plots with an extremely elevated phase angle when on no CPAP. This does not normalize until the ventilator support is increased to PS of 16 over Positive End Expiratory Pressure PEEP of 6.

Figure 15 depicts, in accordance with an embodiment of the invention, the primary analysis which demonstrated that effort of breathing before extubation on CPAP was lower than all other conditions (p = .002). However, there is no difference in effort of breathing between HFNC and NIMV, regardless of flow rate or inspiratory pressure (KW ANOVA p= 0.15).

Figure 16 depicts, in accordance with an embodiment of the invention, the secondary analysis which demonstrated that patients who achieved more synchrony had a larger reduction in effort of breathing on NIMV compared to HFNC (NIMV12 vs HFNC6 and Neo12 vs HFNC6). (R= -0.28, p=0.022, $r^2$ = 0.0778). NIMV results in lower effort of breathing than HFNC when synchrony exceeds 60%.

Figure 17 depicts, in accordance with an embodiment of the invention, that in the 26 patients who had the addition of NeoSeal, synchrony improved from 50% to 55% (p = 0.04

Figure 18 depicts, in accordance with an embodiment of the invention, that of the subset of patients (N=26) who received NeoSeal, there is no difference in effort of breathing regardless of the addition of the nasal clip (p = 0.26).

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within scope of the invention as defined by the appended claims.

## DETAILED DESCRIPTION

It should be understood that this invention is not limited to the particular methodology, protocols, etc., described herein and as such may vary. The terminology used herein is for the purpose of describing particular embodiments only, and is not intended to limit the scope of the present invention, which is defined solely by the claims. As used herein and in the claims, the singular forms include the plural reference and vice versa unless the context clearly indicates otherwise. Other than in the operating examples, or where otherwise indicated, all numbers expressing quantities used herein should be understood as modified in all instances by the term "about."

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments of the aspects described herein, and are not intended to limit the claimed invention, because the scope of the invention is limited only by the claims. Further, unless otherwise required by context, singular terms shall include pluralities and plural terms shall include the singular.

While this invention is susceptible of embodiment in many different forms, there is shown in the drawings and will herein be described in detail preferred embodiments of the invention with the understanding that the present disclosure is to be considered as an exemplification of the principles of the invention and is not intended to limit the broad aspect of the invention to the embodiments illustrated. For purposes of the present detailed description, the singular includes the plural and vice versa (unless specifically disclaimed); the words "and" and "or" shall be both conjunctive and disjunctive; the word "all" means "any and all"; the word "any" means "any and all"; and the word "including" means "including without limitation." Additionally, the singular terms "a," "an," and "the" include plural referents unless context clearly indicates otherwise.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of" refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

Other than in the operating examples, or where otherwise indicated, all numbers expressing quantities used herein should be understood as modified in all instances by the term "about." The term "about" when used in connection with percentages may mean ±1%.

The singular terms "a," "an," and "the" include plural referents unless context clearly indicates otherwise. Similarly, the word "or" is intended to include "and" unless the context clearly indicates otherwise. Thus for example, references to "the method" includes one or more methods, and/or steps of the type described herein and/or which will become apparent to those persons skilled in the art upon reading this disclosure and so forth.

Although methods and materials similar or equivalent to those described herein can be used in the practice or testing of this disclosure, suitable methods and materials are described below. The term "comprises" means "includes." The abbreviation, "e.g." is derived from the Latin exempli gratia, and is used herein to indicate a non-limiting example. Thus, the abbreviation "e.g." is synonymous with the term "for example."

As used herein, a "subject" means a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. Patient or subject includes any subset of the foregoing, e.g., all of the above, but excluding one or more groups or species such as humans, primates or rodents. In certain embodiments of the aspects described herein, the subject is a mammal, e.g., a primate, e.g., a human. The terms, "patient" and "subject" are used interchangeably herein. The human subject may be an adult or a child or an infant.

In some embodiments, the subject is a mammal. The mammal can be a human, non-human primate, mouse, rat, dog, cat, horse, or cow, but are not limited to these examples. Mammals other than humans can be advantageously used as subjects that represent animal models of disorders.

"Esophageal manometry" as used herein refers to a test used to measure the pressure in the lower third of the esophagus, serving as a surrogate for pleural pressure. The result of this test provides the esophageal pressure ($P_{es}$).

"Respiratory inductance plethysmography" (RIP) as used herein refers to a method of evaluating pulmonary ventilation by measuring the movement of the chest (RC) and abdominal (Abd) walls.

"Respiratory rate" refers to the number of breaths per minute. A non-specific measure of respiratory disorder.

"Tidal volume" (Vt) refers to the volume inspired and expired with each breath.

"Minute ventilation" is equivalent to tidal volume multiplied by respiratory rate.

"Work of breathing" is the energy expenditure necessary to accomplish the act of breathing, calculated as the area under the curve of a pressure volume loop during inspiration and/or expiration. It represents lung compliance work, tissue resistance work, airway resistance work, and imposed work from the mechanical ventilator device. When patients are on mechanical ventilators, the "work of breathing" is shared between the patient and the ventilator device.

"Effort of breathing" refers specifically to the patient's expenditure during the process of breathing (not shared with the ventilator). According to a preferred embodiment of the present disclosure, effort of breathing is measured by Pressure-Rate-Product (PRP), although effort of breathing may also be measured by Pressure-Time-Product (PTP) or other measures.

"Respiratory trigger" or "trigger" as used herein refers to detection of the beginning of a breath in (inspiration) and/or out (expiration), so that the start of inspiration and/or expiration may be used for additional processing. As explained further below, triggers are determined by analysis of the real-time signal, by, for example, checking for an incremental increase or decrease within a set time interval. Subsequently, the real-time signal is delayed with the set interval, to synchronize the triggers with the signals and process the signals to calculate all trigger based parameters.

To the extent not already indicated, it will be understood by those of ordinary skill in the art that any one of the various embodiments herein described and illustrated may be further modified to incorporate features shown in any of the other embodiments disclosed herein.

The present technique integrates signals from two sensors to objectively measure the degree of post-extubation UAO. The present technique may also be applied to other aspects of pediatric UAO, including sleep apnea or congenital anomalies such as trachea or laryngomalacia, and anatomically small airways such as those seen with genetic syndromes. In an embodiment, an esophageal balloon catheter is placed through the nose, into the lower 1/3 of the esophagus to obtain esophageal manometry signals, measuring the esophageal pressure to assess the effort of breathing. A second sensor is respiratory inductance plethysmography (RIP), which measures the excursion of the ribcage and the abdomen through elastic bands (like a belt) on the ribcage and abdomen. Using the software modules described herein, the RIP signals are calibrated and integrated with real-time esophageal signals to display flow pressure loops in real time. That information may be used to provide automated interpretation to physicians to definitively diagnose UAO and its severity, but such a step does not form part of the claimed invention.

## Computer-implemented methods

Disclosed herein is a computer method, system, and software for accurately calibrating respiratory inductance plethysmography signals, which when combined with esophageal manometry signals, can determine inspiratory flow limitation and measure effort and/or work of breathing before and after extubation of a patient. According to some embodiments, the system can provide an automated real-time interpretation for the clinician of whether or not the patient has upper airway obstruction, and its relative severity.

Additionally, according to some embodiments, similar methods may be applied to measure effort of breathing (such as from persistent respiratory disease), neuromuscular weakness, or other potential causes of extubation failure. Alternatively, the methods disclosed herein can be applied to the process of weaning a patient from ventilation.

The present inventors have developed a software module, which incorporates the appropriate methods for accurate calibration of RIP signals, and integrates it with real-time esophageal pressure signals. These are displayed in real time as flow-pressure loops that can definitively diagnose UAO. In addition, the present inventors have developed a method, which incorporates the calibrated signals and the effort of breathing of the patient, to provide an automated interpretation for the clinician of whether or not a patient has UAO, and its severity. Such a self-contained system, which can be used in real-time at the patient bedside, did not previously exist for at least pediatric UAO.

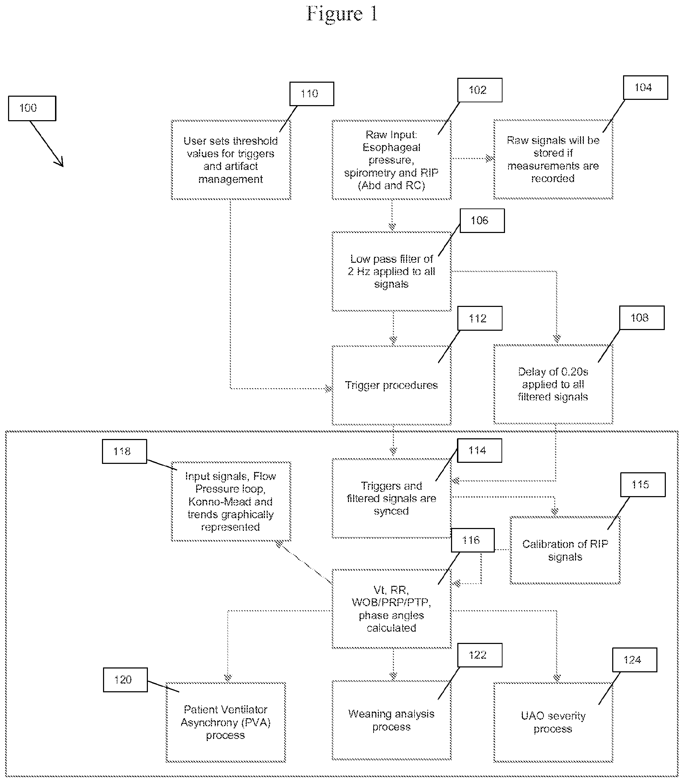

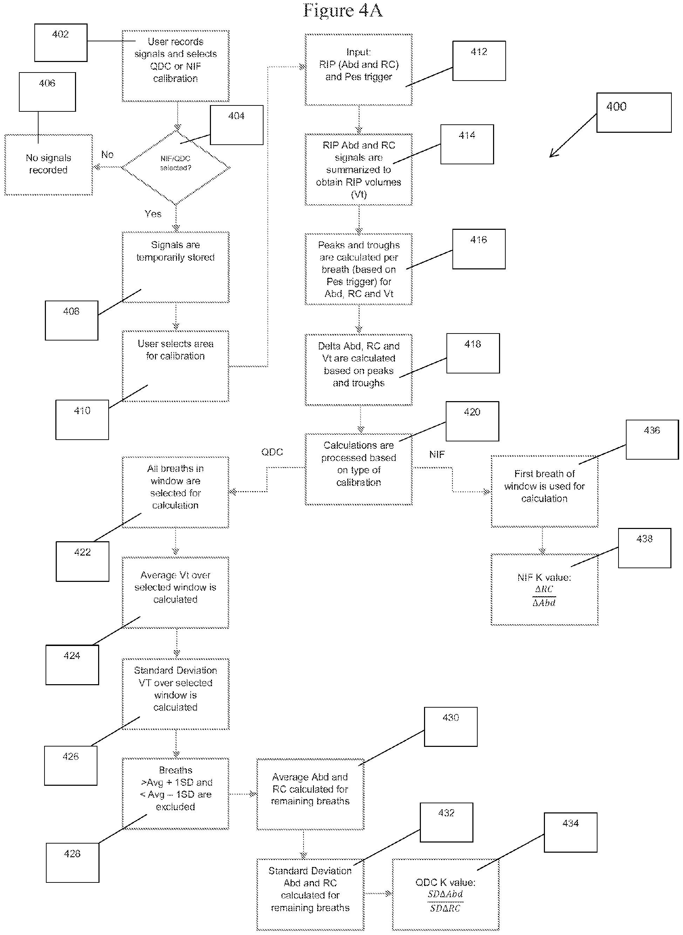

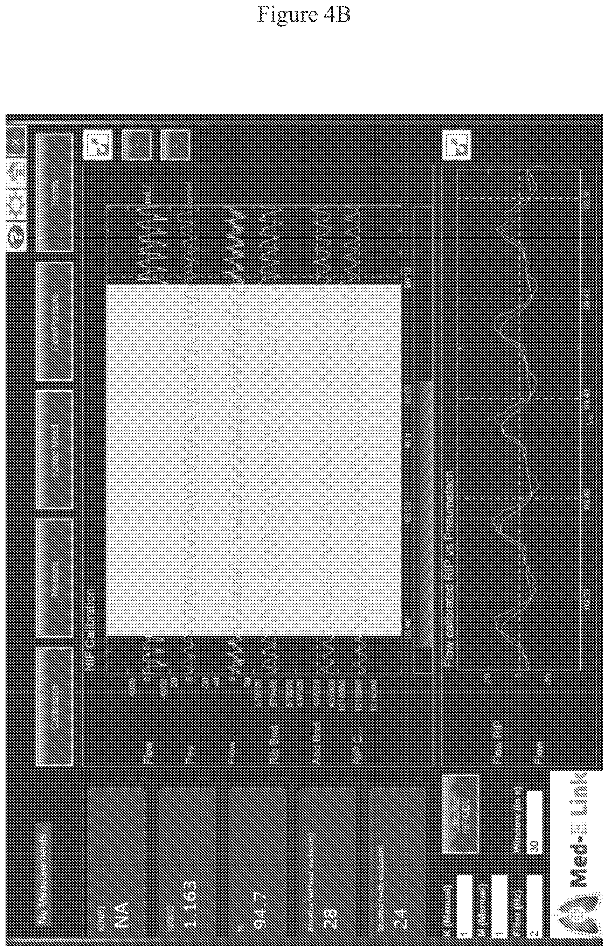

Referring now to FIG. 1, a diagram 100 providing an overview of the effort of breathing assessment process is provided. At 102, input is captured from the patient by measuring the esophageal pressure, spirometry, and/or RIP at both the abdomen and ribcage. At 104, the inputs may be saved for later retrieval if selected by the user. At 106, the inputs are filtered using a 2Hz low pass filter. At 108, the filtered signal is delayed by 0.2 seconds to allow for synchronization with triggers. Additionally, to determine the most accurate measurements, the proper triggers are determined using the processes further detailed below. The trigger process begins by a user selection of the threshold values for triggers and artifacts 110. This input is then used in the trigger process 112. At 114, the triggers are then synched with the filtered and time-delayed signals. At 115, the RIP signals are calibrated as explained below in relation to FIG. 4.

At 116, the system determines Tidal Volume (Vt), Respiratory Rate (RR), Work of Breathing (WOB), Pressure Rate Product (PRP), Pressure Time Product (PTP), and/or phase angles for the current signal as needed for the appropriate display and/or process selected.

At 118, the input data, measurements, and trends may be displayed via a graphical user interface (GUI). Thus, the display may show a user, such as an attending physician, a variety of status information in near real-time. Such display may include a measurement display, with the trigger settings, the filtered signals with time delay, WOB, PRP, PTP, Vt, RR, and/or phase angles. A display of a Konno-Mead plot for the RIP input signals may also be provided. In some embodiments, flow pressure loops for RIP flow or, alternatively, spirometry, against esophageal manometry is provided. Also, trends in WOB, PRP, PTP, RR, Vt, or Patient Ventilator Asynchrony (PVA) may be displayed. Additionally, a score or stop light report, as explained further below in association with analysis methods 120-124, for effort of breathing may be displayed at 118.

Additionally, the user may select from one or more methods of further analyzing potential breathing limitations for the patient. At 120, the PVA method may be performed. The PVA method determines the percentage of patient efforts which result in a ventilator (invasive or non-invasive) supported breath. Patient effort is determined by the esophageal trigger, RIP bands, or a combination thereof. The ventilator delivered breath is determined using spirometry. A breath is considered synchronous if the patient effort results in a ventilator delivered breath within a certain pre-defined time window, for example 0.1, 0.2, or 0.3 seconds.

At 122, the weaning analysis may be performed. And at 124, the UAO severity may be evaluated. According to some embodiments, additional methods of analyzing and reporting measures relating to breathing effort may also be performed. The results of each process may be displayed via the GUI, and may update in near real-time as additional signals are received.

Thus as detailed herein, the software process provides a relative assessment of the patient's breathing capability, which may be used by the attending physician to direct treatment. In the context of the weaning analysis 122, if the patient is being weaned from the ventilator, and if the severity indication shows a high effort of breathing then the physician may therefore discontinue the weaning process and instead increase ventilator support. Otherwise, if the severity indicator shows a low effort of breathing, the weaning process may continue. The physician may continue to monitor the patient using the software methods herein and adjust treatment accordingly.

If the patient has been removed from ventilation, the UAO analysis process 124 may be used to monitor for the presence of UAO. If the UAO severity indicator is high, the attending physician may opt for interventions such as racemic epinephrine, corticosteroids, NIV, or re-intubation.

Depending on the elected treatment, the patient can continue to be monitored for weaning (in the case of non-invasive ventilation) or UAO severity (in the case of post-extubation monitoring and/or intervention) and the attending physician can continue to adjust treatment accordingly based on the reported severity, with the ultimate goal of successfully dismissing the patient from ventilation when appropriate based on the effort of breathing information.

Next, exemplary trigger procedures 112 will be explained in further detail. The software platform enables important feedback loops to accurately calculate the moment of inspiration and expiration for a range of signals such as spirometry, esophageal pressure and RIP. According to some embodiments, the features may include: implementation of a delay of 200ms for all trigger procedures, artifact management, set and reset levels, inspiratory triggers have to be followed by an expiratory trigger, and maximum respiratory rate.

A delay of 200ms can be justified, as the end-user is even unable to observe this delay, and the delay of 200ms enables highly accurate trigger processes. Patients usually do not have inspiratory times exceeding 0.8s, meaning approximately 25% or more of the inspiration has occurred within the set delay. According to some embodiments, a shorter or longer time delay may be used for signal processing. Within this 200ms interval (or other selected time delay interval) the process can verify certain prerequisites for the trigger calculation, such as if a certain incline/decline is met within the timing interval, resulting in a trigger if true and no trigger if false. Trigger levels, for all signals, can be manually adjusted in the module, enabling triggering on an individual basis, giving the best possible calculated parameters for the individual patient.

Patients, especially pediatric patients, do not tend to breathe very regularly, especially on supportive modes of the ventilator. Additionally, physical obstruction in the ventilator system, such as secretions may result in very noisy signals. In order to continuously calculate accurate parameters artifact management is an important task. If applied correctly, it will even ensure extremely precise triggers.

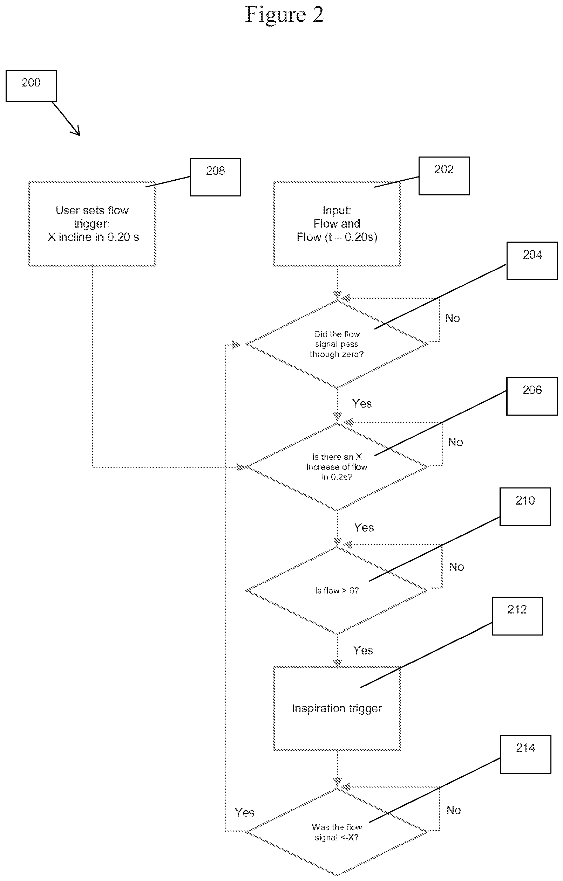

Turning to FIG. 2, a method for determining the flow trigger 200 is detailed. The process starts at 202, with the current flow and the flow detected at 0.2 seconds before the current time are provided as input to the process. According to some embodiments, alternative time delays, such as 0.1 seconds, 0.3 seconds, or other time periods may be used. According to still other embodiments, the time delay may be staggered as to introduce multiple smaller delays, for example two delays of 0.1 seconds may be used to generate the total delay of 0.2. The input signals are updated over time as the current flow data is received. At 204, a check is made to see if the flow passed through zero. If not, the flow continues to be monitored until the check detects the flow signal passed through zero. Once it does, the process continues to 206, where a check is made as to whether the flow increased by a user-selected amount X in the 0.2 second interval. The value of X has been previously selected by the user at 208 and allows the user to adjust the sensitivity of the trigger. Once at least an X increase in flow over the 0.2 second interval is detected, the process continues to 210. At 210, the flow must be greater than zero for the process to continue to 212; otherwise, the flow is continually checked until this condition is met. At 212, the current inspiration trigger has been identified and can be used in the processes as described in 114 and thereon. Therefore, according to the method 200, all prerequisites (flow > 0, increment of X within 0.2 seconds and an inspiration follows expiration) have to be met in order for an inspiration trigger to occur.

At 214, the process repeatedly checks for a flow less than negative X, and once found, the process proceeds back to 204 to detect the zero point. Thus, using the flow trigger process 200 the system can monitor the breathing process and determine the trigger for inspiration for successive breaths, and use the information accordingly for display and further analysis. Thus, the method 200 is able to ignore flow signals rapidly fluctuating around zero (for example, in patients with long expiration periods and secretions) and only include true respiratory efforts, based on the X value selected by the end user, as triggers for further processing.

A trigger essentially demarks the period of inspiration or expiration based on the preconditions described in the flow diagrams. According to some embodiments, triggers can be used to set a window for a calculation (e.g. integration of the area under the pressure volume curve during inspiration to obtain WOB) or be used as a 'one shot' to obtain a value exactly at the moment the signal triggers (e.g. the values of esophageal pressure exactly at the moment of inspiration, which is PEEP). Also, according to some embodiments, inspiratory tidal volumes can be obtained by integrating flow during inspiration.

Table 1 shows an exemplary trigger determination for an example flow signal.

As discussed above, there are several feedback loops within the process to ensure a trigger exactly at 0.00 L/min without false triggers. An initial trigger is fired when the flow signal passes zero, subsequently the set level (an increase of flow in 200ms set by the end-user) has to be met, to validate this trigger. For artifact management purposes a next inspiratory trigger can only be fired when the signals passes the reset level (in the preferred embodiment, the negative flow check of 214). An expiratory trigger can only follow an inspiratory trigger. All these criteria have to be met in order to truly fire a trigger, which is then used for later calculations in the module. This process results in a spirometry trigger at zero flow, working extremely well, even for highly agitated patients with potentially very noisy signals. The same principles may also be applied for esophageal and RIP signals, resulting in very accurate triggers.

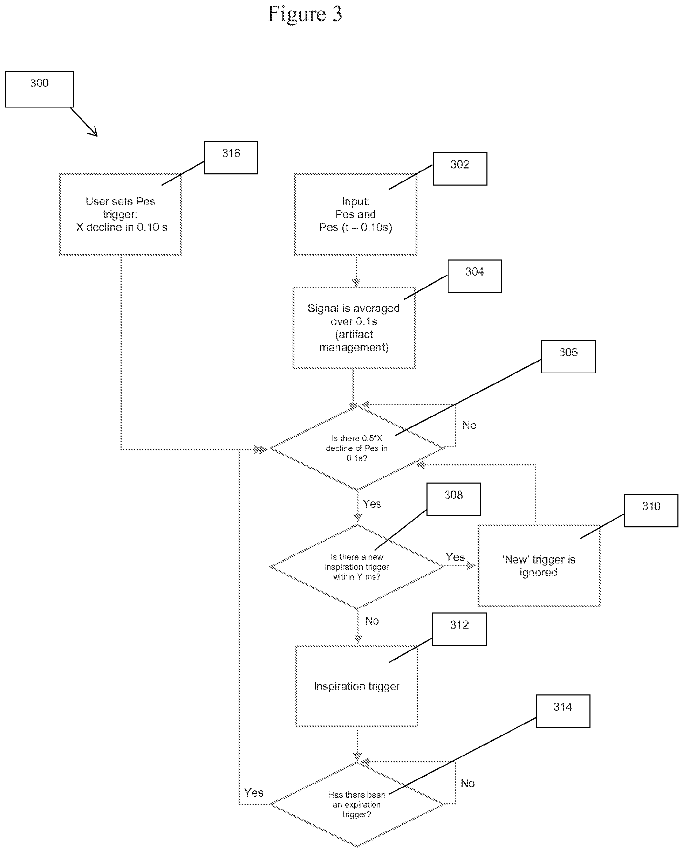

Turning to FIG. 3, a flowchart 300 for identifying triggers within the esopheageal pressure (Pes) signal is provided. At 302, the input of the current Pes and time delayed Pes signal is provided. In this example, the time delay used is 0.1 seconds, although other time delays may be used for the signal. At 304, the Pes signal is averaged over the 0.1 second time delay period to reduce signal artifacts. At 306, a check is performed to determine if there has been a decline in the Pes of 0.5X over the 0.1 second period, where X is the trigger sensitivity parameter entered by the user at 316 (such that higher values of X lead to a less sensitive Pes trigger). If there is not at least a 0.5X decline, the process remains at 306 and continues to monitor incoming Pes signals for that decline. When a decline of at least 0.5X is detected, the process continues to 308 where a check is made to determine if a new inspiration trigger has occurred within Y milliseconds, where Y is a value set by the user based on the RR, in order to exclude unlikely (unreasonably high) values. If a new trigger has occurred within Y milliseconds, this "new" trigger is ignored, as this is likely a false trigger, and the process returns to 306 to check for the next possible trigger. If there has not been a new trigger within Y milliseconds, the current trigger is captured at 312 as the inspiration trigger. At 314, a check is made for an expiration trigger, and this check is continued until an expiration trigger is detected, upon which the process then returns to 306 again. Thus, the process 300 can detect the inspiration points in the signal, while avoiding potential intermittent noise in the Pes signal.

Turning to FIG. 4, an exemplary calibration process 400 for either NIF or QDC calibration is provided. Note that, according to other embodiments, calibration may be performed by least squares analysis.

Calibration is required for the RIP signals. It is used to allow RIP to be representative of flow, particularly after extubation. To do this, the relative contribution of the two RIP bands must be scaled against one another so that when summed they provide a signal proportional to tidal volume (normally measured with spirometry), and then when the derivative of this is taken, a signal proportional to flow is provided (again normally measured with spirometry). The spirometer is removed at extubation, and the calibrated RIP may take its place. The calibrated RIP signal is therefore useful to perform the UAO severity process 124.

To begin the calibration process, at 402 a user selects either QDC or NIF calibration. The user may also desire to record signals and related measurements prior to extubation if desired. At 404, the method checks if either QDC or NIF calibration was selected, and if not, at 406, the calibration process stops and no signals are recorded. Otherwise, at 408, the current signals are temporarily stored in memory and shown on the display, so that at 410 the user may select the area for calibration on the display, as shown in FIG. 4b. In FIG. 4b, the highlighted area represents the selected period used for calibration, in an exemplary embodiment using the Polybench application.

An appropriate breath for NIF calibration, characterized by negative deflection in esophageal pressure, both compartments being ~ 180 degrees out of phase and zero flow measured by spirometry, is manually selected in the reviewer by the user. QDC calibration is applied by selecting an appropriate window with seemingly steady tidal breathing on the reviewer after measurements have been taken.

After selection of an appropriate breathing window for NIF or QDC the process proceeds to 412 where the RIP inputs (for abdomen and ribcage) and Pes trigger are received. At 414, the RIP signals are summed to obtain Vt. Then, at 416, the peaks and troughs for each breath are determined, using the Pes trigger. At 418, the change in the abdomen and ribcage measurements, and the change in the Vt are determined using the peaks and troughs.

At 420, the process proceeds based on the type of calibration selected. If QDC calibration has been selected (at 402), then the process proceeds to 422. If NIF calibration is selected, the process proceeds to 436. Further detail of each type of calibration is provided below.

**NIF Calibration**

At 436, the first breath in the selected window is used for the NIF calibration. The NIF calibration proceeds to determine the K value, based on the delta values for the ribcage and abdomen, which is later used as input to the UAO severity determination at 604.

During obstructive breathing air will get forced from one compartment to the other, the rib cage and abdomen respectively. Equal and opposite contribution of both compartments should therefore result to zero flow in the airways, hence the following model applies:

$$\Delta V_{ao} \cong \Delta V_{RC} + \Delta V_{AB} \qquad \text{Eq. 1}$$

where $\Delta V_{ao}$ is the change of volume measured at the airway by spirometry, $\Delta V_{RC}$ is the change in ribcage volume, and $\Delta V_{AB}$ the change in abdominal volume measured by a calibrated RIP device. This calculation is an approximation, whereas either compartment can move with additional degrees of freedom.

Taking into account the differences in volume motion of both compartments, the following equation sets the changes in uncalibrated rib cage ($\Delta u V_{RC}$) and uncalibrated abdominal ($\Delta u V_{AB}$) volumes approximately equal to $\Delta V_{ao}$ as follows:

$$\Delta V_{ao} \cong M[\Delta u V_{RC} + K(\Delta u V_{AB})] \qquad \textbf{Eq. 2}$$

The term K in equation 2 establishes the electrical proportional relationship between the uncalibrated rib cage ($\Delta u V_{RC}$) and abdominal ($\Delta u V_{AB}$) signals from RIP. K scales both $\Delta u V_{RC}$ and $\Delta u V_{AB}$ to calibrated signals because it adjusts the ratio of the two signals. The term M scales the quantity of $[K(\Delta u V_{RC}) + \Delta u V_{AB}]$ to $\Delta V_{ao}$ as measured with spirometry. As previously stated, the isovolume maneuver causes $\Delta V_{ao}$ to be zero and establishes the following relationship

$$K(\Delta u V_{AB}) \cong -\Delta u V_{RC} \qquad \textbf{Eq. 3}$$

$$K \cong -\frac{\Delta u V_{RC}}{\Delta u V_{AB}} \qquad \textbf{Eq. 4}$$

Showing that when the volume signals from RIP are correctly proportioned in terms of electrical amplifier gains of the device (imbedded wires in the elastic bands) and the volume-motion properties of the two compartments, the change of volume measured in the one compartment is equal to the opposite sign of the volume measured in the other compartment.

This calibration can be applied on the patient's bedside by selecting appropriate breaths in a reviewer after the NIF maneuver. An appropriate breath is characterized by negative deflection in esophageal pressure, both compartments being ~ 180 degrees out of phase and zero flow measured by spirometry. When all conditions are met, the breath(s) can be selected in the reviewer to automatically calculate the calibration factor. Subsequently, the calculated calibration factor will be applied to the abdominal signal which results in a calibrated RIP signal.

**Qualitative Diagnostics Calibration (QDC)**

The QDC algorithm is used to weight the relative gains of each respiratory compartment, typically during tidal breathing. In adults the recommended duration of QDC is five minutes, however it is possible, should it be necessary, to calibrate over shorter periods of time. Children who breathe much faster, probably need shorter periods of calibration, since it is likely the number of breaths is what is important, not the length of time.

Sackner et al. described how the QDC algorithm should be applied to RIP measurements:

$$K_{QDC} = -\frac{SD(\Delta u V_{AB})}{SD(\Delta u V_{RC})} \qquad \textbf{Eq. 5}$$

The calculation is based on the same principles as the isovolume maneuver and thus needs a period of constant tidal breathing to perform this calibration over. However, it is not technically possible for naturally breathing subjects to breath at a constant $V_T$. Collection of a large number of breaths with the exclusion of those with large deviations ($\geq 1$ SD) from the mean sum (of RIP), is therefore used to provide an approximation for $V_T$ to solve equation 5.

Based on the above, the QDC calibration proceeds by selecting the set of breaths in the selected window at 422. At 424, the average Vt is calculated for the breaths in the window, and at 426 the standard deviation for Vt over the window is determined. Then, at 428 the breaths outside of +/- 1 standard deviation are excluded from the analysis. At 430 the remaining breaths are used to determine the average abdomen and ribcage values. The standard deviation for abdomen and ribcage are then calculated for the remaining breaths at 432. Finally, the K value is determined at 434 using the output of 432.

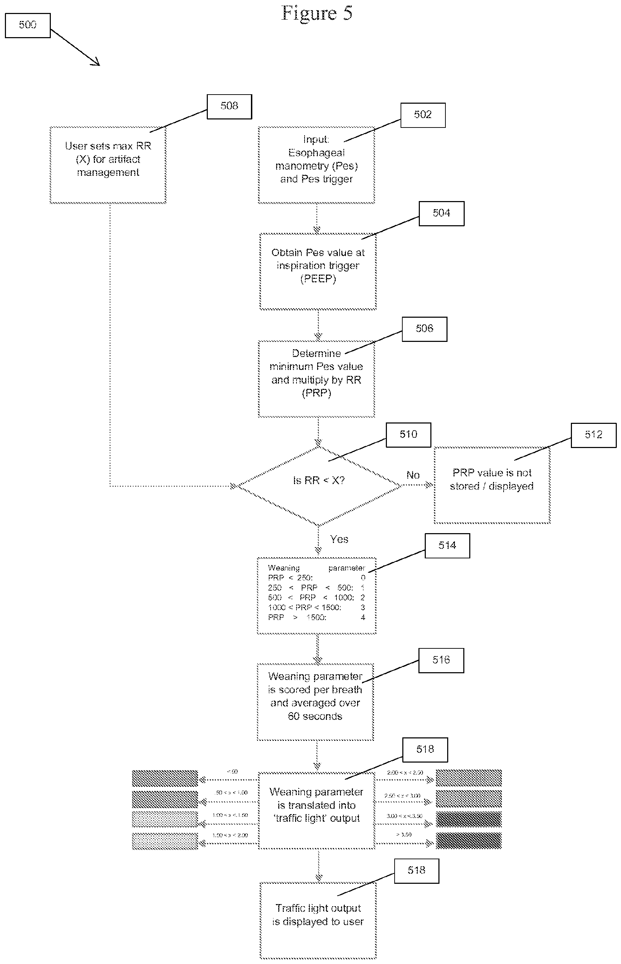

Turning to FIG. 5, an exemplary method 500 for performing weaning analysis and reporting the current severity is provided. According to a preferred embodiment, the weaning parameter is based on effort of breathing using the PRP. Alternatively, the weaning parameter can additionally consider WOB, pressure-time-product, phase angles, and other data in combination with the effort of breathing to determine the severity.

At 502, the input for this process is based on esophageal pressure; the Pes signal and Pes trigger. At 504, the baseline or peak Pes value (PEEP) is obtained at the moment of inspiration, and the minimum or trough value is obtained with a peak/trough detector as discussed above.

At 506, the PRP is determined, by multiplying the change in the esophageal pressure multiplied by the respiratory rate (RR). The respiratory rate is determined by counting the samples between two triggers, which can be translated in to a time (the respiratory cycle time), and then dividing 60 by this value to obtain the respiratory rate.

At 508, the user selects a maximum RR value (X) to be used for artifact management. The selection of this value allows the user to configure the sensitivity of the process. At 510, the value X is compared to the RR value, and if RR is not greater than or equal to X, the current PRP value is discarded and not displayed to the user at 512. Otherwise, if RR is greater than or equal to X, the process proceeds to 514, where the PRP is evaluated based on a relative scoring system. In this embodiment, a PRP below 250 is scored 0, PRP between 250 and 500 is scored a 1, a PRP between 500 and 1000 is scored a 2, and PRP from 1000 to 1500 is scored a 3, and > 1500 is scored a 4. The scoring values, and stop light report values of 518, were selected based on analysis of the processed data from detailed patient studies. At 516, the process continues to score the PRP values and averages the values collected for each breath over a 60 second period. At 518, the average value is evaluated against the traffic light indicator ranges, and the corresponding traffic light and PRP value (and, in some embodiments, the PRP trend) are displayed to the user. According to some embodiments, each traffic light color contains a low and high value, for example, average values between 1.00 and 1.50 would be reported "low yellow" and average values between 1.5 and 2.0 would be reported "high yellow."

According to an alternative embodiment, the average severity is evaluated with regard to the relative change from a baseline value, wherein a significant change in the average severity is indicative of increased effort of breathing, and therefore weaning is contraindicated. According to some embodiments, the traffic light report may be used to signal the relative change in the average severity value.

The PRP is an effective measure of effort of breathing because PRP has an implication for the condition of the patient as it reflects its respiratory condition. During each breath we enlarge the volume of our lungs, by contracting our diaphragm, thereby creating a negative pressure, allowing air to move in. As respiratory pathologies emerge, the exchange of oxygen and carbon dioxide worsens and the patient compensates by enlarging the influx of air by creating more negative pressure and increasing the respiratory rate. An example of different breathing patters is shown in Table 2 (in this case the respiratory pathology is UAO, however other pathologies might result in increased PRP without having flow limitation).

Table 2. Esophageal pressure tracings for a child with UAO from croup (blue tracings) and the same child after administration of racemic epinephrine to treat UAO (pink tracings). Notice the drop in delta esophageal pressure, the reduction in respiratory rate, and overall reduction in pressure rate product (RR*delta esophageal pressure)

In Table 2, note the increased delta pressure and respiratory rate for the patient with UAO. During mechanical ventilation the ventilator takes over some (or all) of the work by forcing positive pressure into the lungs of the patient. As a result the patient has to deliver less work, which is reflected in smaller delta pressures and decreased respiratory rate (i.e. lower PRP). Weaning is the delicate process of getting the patient of the ventilator, by decreasing the supportive pressures of the ventilator. Often ventilators are set at high pressures for long periods of time, because it's impossible to assess the condition of the patient (i.e. protective strategies). This may result in respiratory muscle weakness or atrophy from prolonged respiratory muscle inactivity. Whenever a spontaneous breathing trial is performed on a patient who has been on long periods of high pressure ventilation, the respiratory muscles might not be up for the task, leading to continued need for mechanical ventilation. Additionally, whenever a patient is strong enough to be weaned, this may not recognized, leading to unnecessary long ventilator times. Because treatment with ventilators is associated with ventilator induced lung injury and ventilator associated pneumonia, which are big risk factors for increased morbidity and mortality, it is desirable to wean a patient as soon as possible and reduce unnecessary ventilation.

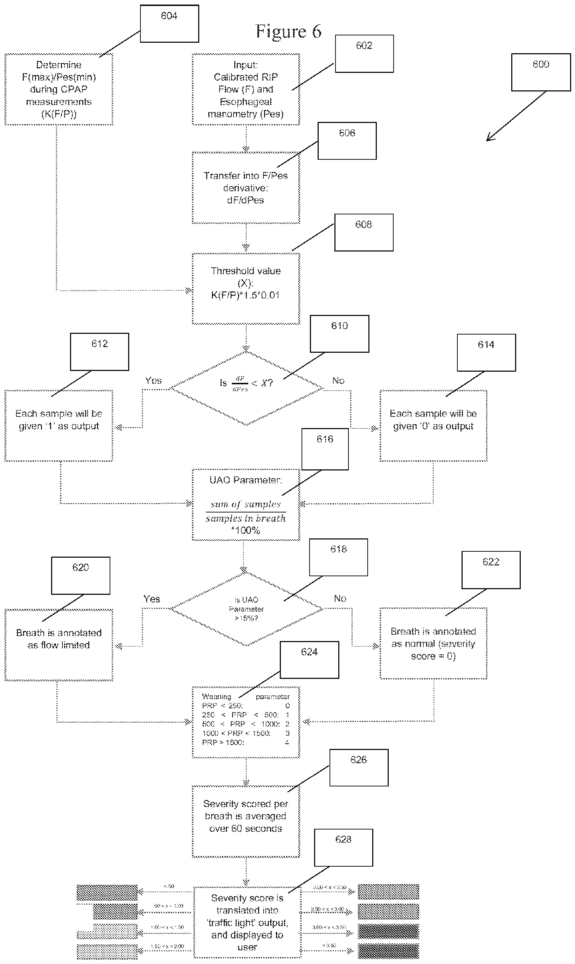

Turning to FIG. 6, a flowchart 600 detailing the process for analyzing and reporting the UAO severity for a patient is provided. At 604, this process starts with the determination of the flow pressure loop slope during CPAP measurements. The calibrated RIP flow signal is plotted against esophageal manometry, to obtain a flow pressure curve. After the calibration process 400, a measurement over several breaths has to be performed, wherein the peak RIP flow is divided by the esophageal pressure value at the moment of inspiration (i.e. PEEP = Positive End Expiratory Pressure). This value (K(F/P)), averaged over a couple of breaths, is used to normalize the data to the patient and to subsequently have a more objective UAO severity parameter. The value K(F/P) is then modified by a constant to determine a threshold value for the comparison at 610. The threshold value is determined in order to objectively distinguish between a normal and flow limited breath. According to some embodiments, and based on current retrospective research, one such value is K(F/P)*1.5*1%, although other threshold values may be used.

At 602, the process receives the inputs of calibrated RIP (thus K is applied to the Abd signal of RIP) and esophageal manometry. At 606, the flow/pressure derivative will be calculated on the fly, resulting in dF/dP dt, which can be compared to the adjusted K(F/P) value at 610. This comparison captures how the derivate (i.e. slope) of the F/P loop behaves over time. In a flow limited breath, a bigger portion of the flow pressure loop will have a smaller slope, as compared to a normal healthy breath.

Thus, during inspiration, the method will continuously check if the criteria dF/dT dt is smaller than K(F/P). If so, at 612, these samples will obtain an '1', and if not, at 614, a '0'. At the end of inspiration all these samples are summarized, divided by the total number of samples during inspiration and multiplied by 100%. This gives the percentage of the breath 616 which was flow limited.

Based on animal and patient data, a threshold value of 15% was selected to label the patient as having inspiratory flow limitation. Thus, a check is performed at 618 to determine if the UAO parameter is greater than 15%. This is the first step to identify UAO severity and is combined with PRP below to report the severity score. If the UAO parameter is not greater than 15%, the breath is not flow limited, and therefore at 622 the severity score for that breath is set to 0. Otherwise, if the UAO parameter is greater than 15%, at 620 the breath is noted as flow limited. According to some embodiments, the threshold value may be adjusted to be higher or lower than 15%.

If the breath is flow limited, then severity is gauged by PRP, which is reported continuously through the software (the calculation of PRP is explained above, in the context of FIG. 5). Data showed a strong correlation between the UAO severity parameter and the subsequent need for re-intubation after extubation. Based on the study data, the severity scores were allocated between selected subgroups of PRP values, as shown at 624. It is understood that these are exemplary values, and may be adjusted based on continued research.

At 626, the UAO severity is collected for a number of breaths for a 60 second period and averaged, as one breath may deviate and not give a good representation of the patient's condition. The 60 second period ensures that there is truly a change in the respiratory condition of the patient in order to change the UAO severity score. According to some embodiments, the average severity may be determined over a longer period, such as 2 minutes or 5 minutes, or a shorter period, such as 30 seconds or 45 seconds, or another time period.

Finally, according to some embodiments, the UAO severity score is translated to a traffic light output (e.g. green = no significant UAO, yellow = mild UAO, orange = moderate UAO, red = severe UAO) and displayed on the GUI. The adequacy of response to treatments for UAO can be gauged by observing changes in UAO severity (such as racemic epinephrine and airway maneuvers or manipulations). In this example, at 628, the possible scores are divided into eight potential gradations of the UAO severity parameter, and can be reported accordingly (e.g. low green, high green, low yellow, high yellow, etc.).

Thus, according to the disclosed methods, the UAO analysis process 124 may be used to monitor for severity of UAO. And if the UAO severity indicator is high, the attending physician may opt for interventions such as racemic epinephrine, corticosteroids, NIV, or re-intubation. Of course, the UAO severity is a recommendation, and the attending physician should use his or her experience and professional opinion to determine the appropriate treatment.

While portions of the disclosure above, for explanatory purposes, discuss methods to process a single breath, it should be understood (except where the context indicates otherwise) that the methods herein are intended to analyze, display, and interpret continuous input signal(s) in real time, by repeating portions of the methods disclosed above as needed (such as through a computer software loop or other mechanism).

Each of the above identified modules, methods, or processes may correspond to a set of instructions for performing a function described above. These modules, methods, or processes need not be implemented as separate software programs, procedures or modules, and thus various subsets of these modules may be combined or otherwise re-arranged in various embodiments. Furthermore, the modules, methods, or processes may be implemented in hardware, firmware, and/or software. In some embodiments, memory may store a subset of the modules and data structures identified above. Furthermore, memory may store additional modules and data structures not described above.

Aspects of the disclosure herein may also be practiced in distributed computing environments where certain tasks are performed by remote processing devices that are linked through a communications network. In a distributed computing environment, program modules can be located in both local and remote memory storage devices.

Moreover, it is to be appreciated that various components described herein can include electrical circuit(s) that can include components and circuitry elements of suitable value in order to implement the embodiments of the subject innovation(s). Furthermore, it can be appreciated that many of the various components can be implemented on one or more integrated circuit (IC) chips, or other hardware or firmware. For example, in one embodiment, a set of components can be implemented in a single IC chip. In other embodiments, one or more of respective components are fabricated or implemented on separate IC chips.

While the present invention has been described with reference to one or more particular embodiments, those skilled in the art will recognize that many changes may be made thereto without departing from the spirit and scope of the present invention. Each of these embodiments and obvious variations thereof is contemplated as falling within the spirit and scope of the invention. It is also contemplated that additional embodiments according to aspects of the present invention may combine any number of features from any of the embodiments described herein.

**Uses of computer-implemented methods**

Provided herein is a method for evaluating medical conditions relating to effort of breathing in a subject in need thereof. The method includes obtaining one or more biometric signals from the subject relating to the subject's breathing; determining a plurality of trigger points for inspiration, based on the one or more biometric signals; calibrating the biometric signals; determining, from the one or more biometric signals, a relative effort of breathing value; and displaying a report comprising an interpretation of the effort of breathing value, wherein the interpretation is updated as additional data relating to the one or more biometric signals is received. In one embodiment, the medical condition being evaluated is the likelihood of extubation failure. In another embodiment, the medical condition being evaluated is UAO and using the methods described herein, wherein UAO is evaluated after extubation. In exemplary embodiments, after extubation may be immediately after extubation, for example, from 5 minutes to 6 hours after extubation. In some embodiments, after extubation is any one or more of 1, 2, 5, 10, 15, 30, 60 90, 120, 150, 180, 210, 240, 270 or 300, 330, 360 or a combination thereof, minutes after extubation. In some embodiments, after extubation includes but is not limited to 7 hours, 8 hours, 9 hours, 10 hours, 11 hours or 12 hours after extubation. In other embodiments, the medical condition being evaluated is persistent respiratory failure, neuromuscular weakness or a combination thereof and using the methods described herein, persistent respiratory failure, neuromuscular weakness or a combination thereof may be evaluated before extubation or after extubation. In various embodiments, before extubation includes but is not limited to immediately prior extubation, or any one or more of 1, 2, 5, 10, 15, 30, 60 90, 120, 150, 180, 210, 240, 270 or 300, 330, 360 minutes or a combination thereof, minutes prior to extubation. In some embodiments, before extubation includes but is not limited to 7 hours, 8 hours, 9 hours, 10 hours, 11 hours or 12 hours before extubation.

In various embodiments, the biometric signals for use in the methods described herein include but are not limited to any one or more of respiratory inductance plethysmography (RIP), esophageal manometry, spirometry or a combination thereof. In various embodiments, the trigger points (for example, as described in Figures 1, 2, 3 and related descriptions set forth herein) for inspiration may delayed by any one or more of 0.05ms, 0.1ms, 0.15ms, 0.2ms, 0.3ms or a combination thereof after inspiration by the subject. In various embodiments, the RIP signal is calibrated while the subject is intubated, prior to extubation, so as to allow for RIP calibration in real-time without processing. In various embodiments, the RIP signal are calibrated using a method including but not limited to continuous positive airway pressure, a quantitative diagnostic calibration (QDC) method, use of negative inspiratory force (NIF) or a combination thereof. In an exemplary embodiment, the calibration process is described in Figure 4 and related description set forth herein.

In various embodiments of the methods described herein, determining the effort of breathing value comprises obtaining the pressure rate product (PRP) and using the PRP values to monitor the need for or changes to medical therapy such as ventilator support. The PRP is obtained by identifying the peak and trough change of the esophageal pressure signals for each breath and multiplying by the respiratory rate (RR). The PRP is calculated over a user defined (pre-determined) period of time of breathing by the subject, which may be any one or more of 10 seconds, 20 seconds, 30 seconds, 40 seconds, 50 seconds, 60 seconds, 90 seconds, 120seconds, 150seconds, 180 seconds, 240 seconds, 300 seconds, 360 seconds, or a combination thereof. For example, the PRP is obtained over 60 seconds of breathing by the subject. In some embodiments, the PRP may be obtained for a longer period of time (for example, by monitoring the subject over a longer time period such as 2-12 hours) but is averaged over smaller windows. As an example, if a patient is monitored for two hours, with preset time intervals of 300s, there will be 24 'average' blocks on the trend viewer, which shows averaged values over the preset time interval. This can be used to monitor the subject's responses to ventilator changes or the development of fatigue. In various embodiments, a severity score is assigned for a given range of PRP values. Merely by way of an example, a severity score of zero may be assigned if the PRP is less than 250; a severity score of one may be assigned if the PRP is between 250 and less than 500; a severity score of two may be assigned if the PRP is between 500 and less than 1000 a severity score of three may be assigned if the PRP is between 1000 and less than 1500, and a severity score of four may be assigned if the PRP is > 1500. The severity scores are then averaged over the number of breaths to obtain a relative averaged severity score. The relative averaged severity score may be displayed in form of, for example, a "traffic light" output (Figure 6). In some embodiments, a normal subject may have a PRP of, for example less than 250 and a relative averaged severity score of 0. In some embodiments, a normal subject may have a relative averaged severity score of less than 1. In some embodiments, instead of a 0-4 severity score, the actual PRP values may be used to determine changes in effort of breathing in response to therapies. Some situations (such as titration of ventilator settings) may warrant using PRP as a continuous variable to determine the optimal level of ventilator support because the granularity of the severity score is inadequate to measure smaller but important relative changes in effort of breathing. In an embodiment, the assessment of UAO severity is described herein and in Figure 6. In other embodiments, the severity scores may be used to guide ventilator weaning, wherein the physician may alter the ventilator support to maintain a patient in a desired range (i.e. between 1 and 2 for weaning range).

In another embodiment, determining the relative effort of breathing and UAO severity value comprises using the change relative to Continuous Positive Airway Pressure (CPAP). Rather than using set ranges for PRP, the UAO severity parameter represents a change in PRP after extubation from the pre-extubation value on CPAP (expressed as post-extubation PRP/CPAP PRP). A range < 1.5 may be considered a severity score of 0, 1.5-2 a severity score of 1, 2-3 a severity score of 2, and > 3 a severity score of 3, for example. The methods of the invention may be practiced as described herein and in Figures 1-6.

Also provided herein is a method for determining the severity of post-extubation UAO in a subject in need thereof. The method includes providing a first and second sensor; obtaining esophageal pressure ($P_{es}$) signals in the subject using the first sensor; obtaining respiratory inductance plethysmography (RIP) signals in the subject using the second sensor; calibrating the RIP signals obtained from the second sensor; determining the presence of inspiratory flow limitation; obtaining a pressure rate product (PRP) for each breath to determine associated relative severity of inspiratory flow; and averaging the relative severity over the number of breaths taken over a period of time, wherein an increase in the relative averaged severity score compared to a normal subject is indicative of increased severity of post-extubation UAO. In some embodiments, the RIP signals are calibrated as described in Figure 4 and related description set forth herein. In an embodiment, the inspiratory flow limitation is determined after extubation as described in Figure 6 and related description set forth herein. The calibrated RIP signals and esophageal pressure signals are used to determine the severity of post-extubation UAO as described in Figure 6 and related description herein set forth herein. In one embodiment, the severity of post-extubation UAO is assessed after extubation. As described herein, a relative averaged severity score is obtained based on the PRP. An increase in the relative averaged severity score in the subject compared to a normal subject is indicative of increased severity of post-extubation UAO. In some embodiments, a normal subject may have a PRP of, for example less than 250 and a relative averaged severity score of 0. In some embodiments, a normal subject may have a relative averaged severity score of less than 1. In an embodiment, the assessment of UAO severity is described herein and in Figure 6. In some embodiments, instead of a 0-4 severity score, a more continuous score may be used as described herein. In exemplary embodiments, after extubation may be immediately after extubation, from 5 minutes to 6 hours. In addition to using PRP values to determine the severity of post-extubation UAO, changes in breathing relative to CPAP as described herein may also be used to determine the severity of post-extubation UAO. In an embodiment, an increase in severity of UAO is indicative of increased likelihood of post-extubation failure.

Further provided herein is a method for determining the likelihood of post-extubation failure in a subject with persistent elevation in effort or work of breathing due to persistent respiratory diseases. The method includes providing a first and optionally, a second sensor; obtaining esophageal pressure ($P_{es}$) signals in the subject using the first sensor; optionally obtaining respiratory inductance plethysmography (RIP) signals in the subject using the second sensor; if obtained, calibrating the RIP signals obtained from the second sensor; determining the presence of inspiratory flow limitation; obtaining a pressure rate product (PRP) for each breath to determine associated relative severity of inspiratory flow; and averaging the relative severity over the number of breaths taken over a period of time, wherein an increase in the relative averaged severity score relative to normal subject is indicative of increased likelihood of post-extubation failure in the subject with persistent elevation in effort or work of breathing due to persistent respiratory diseases. In an embodiment, the subject with persistent elevation in effort or work of breathing due to persistent respiratory diseases does not have UAO. In some embodiments, the RIP signals are calibrated as described in Figure 4 and related description set forth herein. In an embodiment, the inspiratory flow limitation is determined before or after extubation as described in Figure 6 and related description set forth herein. The calibrated RIP signals and esophageal pressure signals are used to determine the likelihood of post-extubation failure in the subject as described in Figure 6 and related description herein set forth herein. As described herein, a relative averaged severity score is obtained based on the PRP while on CPAP. An increase in the relative averaged severity score in the subject relative to a normal subject is indicative of increased likelihood of post-extubation failure in the subject with persistent elevation in effort or work of breathing due to persistent respiratory diseases. In some embodiments, a normal subject may have a PRP of, for example less than 250 and a relative averaged severity score of 0. In some embodiments, a normal subject may have a relative averaged severity score of less than 1. In some embodiments, instead of a 0-3 severity score, a more continuous score may be used as described herein. In exemplary embodiments, after extubation may be immediately after extubation, from 5 minutes to 12 hours. In exemplary embodiments, before extubation may be immediately before extubation, within 6 hours. In addition to using PRP values to determine the likelihood of post-extubation failure in the subject with persistent elevation in effort or work of breathing due to persistent respiratory diseases, changes in breathing relative to CPAP as described herein may also be used to determine the likelihood of post-extubation failure in the subject with persistent elevation in effort or work of breathing due to persistent respiratory diseases. In some embodiments, persistent respiratory diseases include but are not limited to pneumonia, acute respiratory distress syndrome, scoliosis, large abdominal compartment, or lower airway obstruction from asthma, bronchiolitis or Chronic Obstructive Pulmonary Disease.

Further provided herein is a method for determining the likelihood of post-extubation failure in a subject with neuromuscular diseases. The method includes providing a first and a second sensor; obtaining esophageal pressure ($P_{es}$) signals in the subject using the first sensor; obtaining respiratory inductance plethysmography (RIP) signals in the subject using the second sensor; calibrating the RIP signals obtained from the second sensor; determining the direction of the Konno-Mead Plot and Phase Angle; obtaining a pressure rate product (PRP) for each breath; and examining the change in PRP and Konno Mead Plots over time on CPAP. Increased phase angle, clockwise direction of the Konno-Mead Plots, Figure of 8 appearances on Konno-Mead, and relatively low values for PRP or worsening of PRP over time on CPAP is indicative of increased likelihood of post-extubation failure in the subject with neuromuscular diseases. In an embodiment, the subject with neuromuscular diseases does not have UAO. In some embodiments, the RIP signals are calibrated as described in Figure 4 and related description set forth herein. In exemplary embodiments, after extubation may be immediately after extubation, from 5 minutes to 48 hours. In exemplary embodiments, before extubation may be immediately before extubation, within 24 hours. In addition to using PRP values to determine the likelihood of post-extubation failure in the subject with neuromuscular diseases, changes in breathing relative to CPAP as described herein may also be used to determine the likelihood of post-extubation failure in the subject with neuromuscular diseases. In some embodiments, neuromuscular diseases include but are not limited to diaphragm atrophy from prolonged ventilation, botulism, spinal cord injury, unilateral or bilateral diaphragm paresis, muscular dystrophy, or other neuromuscular conditions.

Critical care practitioners increasingly use non-invasive modes of mechanical ventilation including humidified high flow nasal cannula [HFNC] and nasal intermittent mechanical ventilation [NIMV], with the goal of reducing work of breathing. It is unclear if NIMV is superior to HFNC at reducing work of breathing, and if patient NIMV synchrony is an important factor in reducing work of breathing. We sought to directly compare effort of breathing on HFNC vs. NIMV for children < 6 months of age using esophageal manometry. We hypothesized that NIMV would result in lower effort of breathing than HFNC when the majority of NIMV breaths were synchronous.

Accordingly, provided herein is a method for assessing synchrony between patient and mechanical ventilators so that as synchrony improves, the effort of breathing in the subject is reduced. In various embodiments, the methods for assessing synchrony (PVA) comprises obtaining esophageal manometry signals from the subject and spirometry signals from the mechanical ventilator device. The PVA method determines the percentage of patient efforts which result in a ventilator (invasive or non -invasive) supported breath. Patient effort is determined by the esophageal trigger, RIP bands, or a combination thereof. The ventilator delivered breath is determined using spirometry. A breath is considered synchronous if the patient effort results in a ventilator delivered breath within a certain pre-defined time window, for example 0.1, 0.2, or 0.3 seconds. Subsequently the pressure rate product (PRP = peak-to-trough change in esophageal pressure * respiratory rate) is determined by the methods described herein. The PRP is calculated over a user defined (pre-determined) period of time of breathing by the subject, which may be any one or more of 10 seconds, 20 seconds, 30 seconds, 40 seconds, 50 seconds, 60 seconds, 90 seconds, 120seconds, 150seconds, 180 seconds, 240 seconds, 300 seconds, 360 seconds, or a combination thereof. For example, the PRP is obtained over 60 seconds of breathing by the subject. In various embodiments, a severity score is assigned for a given range of PRP values. Simply by way of an example, a severity score of zero may be assigned if the PRP is less than 250; a severity score of one may be assigned if the PRP is between 250 and less than 500; a severity score of two may be assigned if the PRP is between 500 and less than 1000 a severity score of three may be assigned if the PRP is between 1000 and less than 1500, and a severity score of 4 may be assigned if the PRP is > 1500. The severity scores are then averaged over the number of breaths to obtain a relative averaged severity score. The relative averaged severity score may be displayed in form of, for example, a "traffic light" output (Figure 6). In some embodiments, a normal subject may have a PRP of, for example less than 250 and a relative averaged severity score of 0. In some embodiments, a normal subject may have a relative averaged severity score of less than 1. In some embodiments, instead of a 0-4 severity score, a more continuous scale with display of PRP may be used as described herein. In some embodiments, alterations of the mechanical ventilator support and triggering mechanisms of the ventilator, sealing air leak, or altering flow patterns of the ventilator will result in higher degrees of synchrony (increase in percentage of PVA) between the ventilator and the patient, and reduce the effort of breathing or the severity score.

In one aspect, provided herein is a computer implemented method for determining whether a subject has a medical condition, comprising: on a device having one or more processors and a memory storing one or more programs for execution by the one or more processors, the one or more programs including instructions for: obtaining a biometric signal from the subject; calibrating the biometric signal to measure a biological function relating to the biometric signal before and after a medical procedure to provide an automated real-time interpretation of whether or not the subject has a problem with the biological function, and a severity of the problem with the biological function; and sending a signal relating to the biometric signal, the biological function, the problem or the severity. In one embodiment, the biometric signal comprises respiratory inductance plethysmography and esophageal manometry signals. In another embodiment, the biological function comprises effort of breathing from esophageal manometry. In some embodiments, the medical procedure comprises extubation of the subject. The problem with the biological function comprises an upper airway obstruction. In some embodiments, the calibration comprises any one or more of calibration of continuous positive airway pressure, a quantitative diagnostic calibration method, a least squares calibration method and use of negative inspiratory force for an intubated subject to calibrate respiratory inductance plethysmography flow prior to extubation so as to allow for calibration in real-time without post-processing. In some embodiments, the subject is an adult human, a human child or a human infant.

In another aspect, provided herein is a computer system for determining whether a subject has a medical condition, comprising: one or more processors; and memory to store: one or more programs, the one or more programs comprising instructions for: obtaining a biometric signal from the subject; calibrating the biometric signal to measure a biological function relating to the biometric signal before and after a medical procedure to provide an automated real-time interpretation of whether or not the subject has a problem with the biological function, and a severity of the problem with the biological function; and sending a signal relating to the biometric signal, the biological function, the problem or the severity. In one embodiment of this aspect, the biometric signal comprises respiratory inductance plethysmography and esophageal manometry signals. In another embodiment, the biological function comprises effort of breathing from esophageal manometry. In a further embodiment, the medical procedure comprises extubation of the subject. In an embodiment, the problem with the biological function comprises an upper airway obstruction. In some embodiments, calibration comprises one from the group consisting of: calibration of continuous positive airway pressure, a quantitative diagnostic calibration method, a least squares calibration method and use of negative inspiratory force for an intubated subject to calibrate respiratory inductance plethysmography flow prior to extubation so as to allow for calibration in real-time without post-processing.

In another aspect, provided herein is a non-transitory computer-readable storage medium storing one or more programs for determining whether a subject has a medical condition, the one or more programs for execution by one or more processors of a computer system, the one or more programs comprising instructions for: obtaining a biometric signal from the subject; calibrating the biometric signal to measure a biological function relating to the biometric signal before and after a medical procedure to provide an automated real-time interpretation of whether or not the subject has a problem with the biological function, and a severity of the problem with the biological function; and sending a signal relating to the biometric signal, the biological function, the problem or the severity. In one embodiment, the biometric signal comprises respiratory inductance plethysmography and esophageal manometry signals.

The biological function comprises effort of breathing from esophageal manometry. In a further embodiment, the medical procedure comprises extubation of the subject. The problem with the biological function comprises an upper airway obstruction. In various embodiments, calibration comprises any one or more of calibration of continuous positive airway pressure, a quantitative diagnostic calibration method, a least squares calibration method and use of negative inspiratory force for an intubated subject to calibrate respiratory inductance plethysmography flow prior to extubation so as to allow for calibration in real-time without post-processing

## EXAMPLES

The following examples illustrate some embodiments and aspects of the invention. It will be apparent to those skilled in the relevant art that various modifications, additions, substitutions, and the like can be performed without altering the spirit or scope of the invention, and such modifications and variations are encompassed within the scope of the invention as defined in the claims, which follow. The following examples do not in any way limit the invention.

## Example 1: Experimental Methods

An interventional trial in intubated and ventilated children admitted to the pediatric or cardiothoracic intensive care units was performed. Children were included if they were between 37 weeks corrected gestational age and 18 years, were intubated for at least 12 hours, and had a planned extubation from 7 am to 5 pm Monday through Friday, when research personnel were available. Children were excluded if they had a contraindication to receive an esophageal catheter (high risk of bleeding, esophageal abnormalities) or Respiratory Inductance Plethysmography bands (abdominal or chest wall defects).

*Study Protocol*

After informed consent, an age appropriate esophageal balloon catheter (Carefusion, Avea SmartCath 6,7, or 8Fr) was placed through the nose to the lower 1/3 of the esophogus, with catheter position either by chest radiograph or monitoring deflections of pressure during brief endotracheal tube occlusions [Coates AL, Davis GM, Vallinis P, Outerbridge EW: Liquid-filled esophageal catheter for measuring pleural pressure in preterm neonates. J Appl Physiol 1989, 67(2):889-893]. Once the clinical team decided to extubate the child, age appropriate RIP bands (Viasys Healthcare, Respiband Plus, Hoechberg, Germany or Nox Medical) were placed around the abdomen and ribcage, and an age appropriate self-calibrating pneumotachometer (Viasys Variflex 51000-40094) was connected to the endotracheal tube. These sensors were attached to the Bicore II hardware device (CareFusion, Houten, The Netherlands). The data were displayed and recorded (at a frequency of 200Hz) on a laptop computer using a custom module developed in Polybench (Applied Biosignals GmbH, Weener, Germany). We then post processed the obtained measurements for all study calculations using a custom module using VivoSense® software (Vivonetics, San Diego, CA, USA).

After verification that all sensors were accurately displaying data, patients underwent 5 minutes of spontaneous breathing on Continuous Positive Airway Pressure (CPAP) of 5cmH20 (without pressure support), and a standardized Negative Inspiratory Force (NIF) [Bagshaw SM, Delaney A, Farrell C, Drummond J, Brindley PG: Best evidence in critical care medicine. Steroids to prevent post-extubation airway obstruction in adult critically ill patients. Can J Anaesth 2008, 55(6):382-385] maneuver. The NIF was performed by disconnecting the patient from the ventilator, adding a pressure manometer distal to the spirometer, and occluding the endotracheal tube for 3-5 consecutive breaths at end exhalation. Prior to extubation, data regarding potential risk factors for post-extubation UAO were gathered including data on endotracheal tube size, the presence of a leak at placement of the endotracheal tube, age, length of mechanical ventilation, Ramsey Sedation Scale at the time of extubation, fluid status, leak at extubation and pressure, cuff leak volume, history of airway trauma or multiple intubation attempts, diagnostic information, and the average of all charted pain scores for the previous 24 hours (as a surrogate for adequacy of sedation). Extubation leak pressure was assessed after suctioning the endotracheal tube, with the head in midline position, and the cuff of the endotracecheal tube completely deflated (when a cuffed tube was in place). The pressure heard audible to the ear (without a stethoscope) was recorded to a maximal pressure of 40 cmH20. In addition, all patients were placed on standard ventilator settings in a Pressure Control mode with a rate from 10-20 bpm (based on age), a Peak Inspiratory Pressure of 20 cmH20, a PEEP of 5 cmH20, and Pressure support of 10cmH20. For uncuffed endotracheal tubes, the difference between inspiratory and expiratory tidal volume divided by inspiratory tidal volume during a ventilator breath was recorded as a leak percentage. For cuffed endotracheal tubes, the cuff leak volume was calculated as the difference between expiratory tidal volume with the cuff inflated versus deflated divided by expiratory tidal volume with the cuff inflated [Chung Y-H, Chao T-Y, Chiu C-T, Lin M-C: The cuff-leak test is a simple tool to verify severe laryngeal edema in patients undergoing long-term mechanical ventilation. Crit Care Med 2006, 34(2):409-414; Cheng KC, Hou CC, Huang HC, Lin SC, Zhang H: Intravenous injection of methylprednisolone reduces the incidence of postextubation stridor in intensive care unit patients. Critical Care Medicine 34(5):1345-50, 2006 May].

After extubation, the spirometer was removed but the esophageal catheter and RIP bands stayed in place. Standardized measurements were made 5 and 60 minutes after extubation. Simultaneously, three bedside providers (nurse (RN), respiratory therapist (RT), and physician (MD)) performed blinded assessments of stridor severity using a scoring sheet based on the Westley Croup score, which we have previously validated [Khemani R, Schneider J, Morzov R, Markovitz B, Newth CJ: Pediatric Upper Airway Obstruction: Interobserver variability is the road to perdition. J Crit Care 2013, 28(4):490-497]. One of the providers was required to have gone through a 2 hour post extubation UAO training session, and was labeled a "core trained" individual. If an intervention was prescribed by the clinical team (such as racemic epinephrine), data was recorded before and after the intervention at standardized time intervals. Research personnel were present for all extubations, and when UAO was suspected through interpretation of data from the simultaneous RIP and esophageal manometry measurements, an Esmark Maneuver was performed in an attempt to identify if the obstruction was likely supraglottic [Arai YC, Fukunaga K, Hirota S, Fujimoto S, Arai Y-CP, Fukunaga K, Hirota S, Fujimoto S: The effects of chin lift and jaw thrust while in the lateral position on stridor score in anesthetized children with adenotonsillar hypertrophy. Anesth Analg, 99(6):1638-1641].

**Example 2: Outcome determination: Upper airway Obstruction**

Post extubation UAO was assessed by examining the combination plot of calibrated Respiratory Inductance Plethysmography as a measure of flow, and esophageal pressure for the presence of inspiratory flow limitation. Inspiratory flow limitation is characterized by a disproportionately high inspiratory effort (negative esophageal pressure) relative to the increase in flow. RIP flow was calibrated under two conditions: while spontaneously breathing on CPAP of 5 prior to extubation using an algorithm called Quantitative Diagnostic Calibration, and during a NIF maneuver prior to extubation as an isovolume maneuver (Sackner, 1989 #319). We have previously demonstrated that NIF calibration (isovolume conditions) was superior to QDC on CPAP, particularly as UAO worsened. For this reason, NIF calibration of RIP was used for the primary outcome of inspiratory flow limitation using the combination of RIP and esophageal manometry. Flow limitation was graded using the combination flow/pressure plots while intubated, as well as 5 and 60 minutes after extubation using a scale from 0-3 initially proposed by Kaplan [Kaplan V, Zhang JN, Russi EW, Bloch KE: Detection of inspiratory flow limitation during sleep by computer assisted respiratory inductive plethysmography. Eur Respir J 2000, 15(3):570-578]. When inspiratory flow limitation was new or worse after extubation (extubation value > than CPAP value), UAO severity was scored (none, mild, moderate, severe) using the relative change in effort of breathing, measured by Pressure.Rate.Product (Respiratory rate * change in esophageal pressure) before and after extubation. For analysis, patients were labeled has having post-extubation UAO when the PRP after extubation was at least 50% higher than CPAP values (minimum mild UAO). When available, data from before and during the Esmark maneuver was used to classify patients whose UAO was likely supraglottic, defined as at least a 50% reduction in effort of breathing during an airway maneuver. Allocation of the primary outcome of UAO was made independently of clinical assessment of UAO. Clinical outcomes such as re-intubation, non-invasive ventilation use, therapies to treat UAO (racemic epinephrine, heliox, corticosteroids) were followed for 48 hours after extubation.

**Example 3: Analysis**

To assess whether the objective characterization of UAO severity was relevant against clinical outcomes, the UAO severity parameter, gauged 5 minutes after extubation, was evaluated against the outcome of re-intubation within 48 hours with an area under the curve (AUC) of the receiver operating characteristic (ROC) plot.

To assess whether the objective parameter was different than clinical assessment of stridor, we collapsed the Westley stridor scores of physicians, nurses, and respiratory therapists into very mild (stridor but no retractions), mild, moderate and severe categories based on the presence of stridor and the severity of gauged retractions. We report the predictive ability of these scores 5 minutes after extubation on the outcome of re-intubation, as well as describe the inter-observer variability between providers when assessing post-extubation UAO 5 and 60 minutes after extubation, as well as the variability between clinical providers and the objective assessment of UAO (using flow limitation algorithm) 5 and 60 minutes after extubation.

We sought to identify risk factors for post-extubation UAO using the flow-limitation assessment as the main outcome, and determine whether the level of extra thoracic obstruction (supraglottic versus glottic/subglottic) was relevant for risk factors and outcomes. Descriptive statistics are reported by group using median (interquartile range) or number (percent), and analyzed with a Mann Whitney U test or Chi-squared test, as appropriate. Test statistics (sensitivity, specificity, likelihood ratios) were computed for leak percentage variables, and AUC of ROC plots were calculated using empirical estimates of the ROC plot with respective 95% Confidence intervals. Statistical comparison of ROC plots was made with a Chi-squared test. A multivariate logistic regression model was build examining risk factors with a univariate association with post-extubation UAO. Statistical analysis was performed in Statistica and stata 10.

**Example 4: Results**

1182 patients were eligible for the study, and 471 were consented. 409 were ultimately included in the final analysis.

The study cohort comprised of 409 patients, with a median age of 5 months (IQR 1, 16) and 55% male. Approximately half of the patients were intubated for cardiac surgery. There were slightly more patients with uncuffed endotracheal tubes (n=240) than cuffed tubes (n=169). Overall, 34 patients were re-intubated (8.3%), and 107 patients (26%) were labeled as having post-extubation UAO based on the presence of inspiratory flow limitation and increased PRP after extubation. The re-intubation rate was 18.7% for those with UAO, and 5.3% for those without UAO. Of the 34 patients who were re-intubated within 48 hours, 20 (58.8%) had post extubation UAO.

*UAO Severity and re-intubation*

Using the UAO severity marker measured 5 minutes after extubation, there is a stepwise increase in both the number of patients and the re-intubation rates when using the flow limitation and effort of breathing algorithm (Table 3). Overall, there was a 23.4% re-intubation rate for those labeled with UAO using the algorithm 5 minutes after extubation. The AUC of the ROC plot for the UAO severity parameter for all cause re-intubation is 0.70 (0.61, 0.79), and for Re-intubation from UAO is 0.91 (95% CI 0.85, 0.97).

Table 3: Flow Limitation algorithm and MD, RN, RT assessment of UAO severity 5 minutes after extubation. UAO severity increases as the number climbs above 0, with 0 indicative of no UAO. Outcome is re-intubation within 48 hours of extubation.

| UAO Severity | UAO Tool | | MD | | RN | | RT | |
|---|---|---|---|---|---|---|---|---|
| | Re -int. (Rate) | Total | Re -int. (Rate) | Total | Re -int. (Rate) | Total | Re -int. (Rate) | Total |
| **0** | 10 (4.7%) | 319 | 23 (7%) | 324 | 17 (5.5%) | 312 | 17 (5.4%) | 313 |
| **1** | 5 (18.5%) | 27 | 1(10%) | 10 | 0 (0%) | 15 | 0 (0%) | 18 |
| **2** | 3 (9%) | 34 | 3 (6.5%) | 46 | 11 (20%) | 55 | 6 (13.6%) | 44 |
| **3** | 5 (28%) | 18 | 3 (18.7%) | 16 | 4 (17%) | 23 | 9 (30%) | 30 |
| **4** | 6 (55%) | 11 | 4 (57%) | 7 | 2 (50%) | 4 | 2 (50%) | 4 |

When using the collapsed Westley Score which bedside providers assessed 5 minutes after extubation, fewer patients are identified as having severe UAO (Table 3). When bedside providers labeled the patient as having UAO, the re-intubation rates were 15.1% (MD), 17.5% (RN), and 17.7% (RT). The UAO severity parameter better discriminated all cause re-intubation compared to MD UAO assessment (AUC 0.58 (0.49, 0.67), p=0.007), and equally well as RN (AUC 0.66 (0.56, 0.75 CI, p=0.4), or RT (0.67 (0.57, 0.76, p=0.4) assessment. The UAO severity parameter better discriminated re-intubation from UAO than MD (AUC 0.69 (0.56, 0.81), p=0.0002), RN (AUC 0.70 (0.58, 0.82 CI, p=0.0001), or RT (0.78 (0.67, 0.91, p=0.01) UAO assessment.

*Variability amongst bedside providers in assessing post extubation UAO*

There is significant variability between providers when trying to identify the presence or absence of post extubation UAO 5 minutes after extubation. For this analysis, post extubation UAO was labeled as stridor with at minimum mild retractions. While the percent agreement ranged from 77-81%, the kappa values were between 0.33-0.43 when comparing Nurses, MDs, and Respiratory Therapists (Table 4) to each other.

Table 4: Percent agreement and inter-rater reliability of presence or absence of post-extubation UAO 5 minutes after extubation between tool and bedside providers (left) and amongst bedside providers (right). For analysis, UAO was labeled as present per bedside providers when the patient had stridor plus at minimum mild retractions (minimum score 2 or greater from Table 3). For the UAO tool, UAO was labeled when the patient had new flow limitation with an increase in effort of breathing 1.5 times the pre-extubation value (minimum score 1 or greater from Table 3.

| | FL_No | FL_Yes | | RN_No | RN_Yes |
|---|---|---|---|---|---|
| MD_No | 290 | 36 | MD_No | 273 | 57 |
| MD_Yes | 40 | 37 | MD_Yes | 33 | 40 |
| 81.1 % Agreement κ =0.38 | | | 77.6% Agreement κ =0.33 | | |
| | FL_No | FL_Yes | | RT_No | RT_Yes |
| RN_No | 276 | 56 | MD_No | 280 | 50 |
| RN_Yes | 36 | 41 | MD_Yes | 27 | 46 |
| 77.5% Agreement κ =0.33 | | | 80.9% Agreement κ =0.43 | | |
| | FL_No | FL_Yes | | RN_No | RN_Yes |
| RT_No | 283 | 49 | RT_No | 269 | 44 |
| RT_Yes | 30 | 47 | RT_Yes | 43 | 53 |
| 80.7% Agreement κ =0.42 | | | 78.7% Agreement κ =0.41 | | |

When comparing the assessments of the bedside providers to the flow limitation algorithm, the percent agreement ranged between 77-81%, with kappa values from 0.33-0.52 (Table 4). When examining the patients who were classified differently by the UAO algorithm and bedside providers (Table 5), in general there were low re-intubation rates (<5%) when the provider and the tool both assess the patient as not having UAO. There are high rates of re-intubation when both provider and tool agree that UAO is present (27-30%). There are low rates of re-intubation when the clinician labels the patient with UAO, but the tool does not (2-9%). There are high rates of re-intubation when the tool detects UAO, but the clinician does not (13-20%).

Table 5: Outcomes and re-intubation rates based upon agreement or disagreement between bedside providers and UAO algorithm. There are high rates of re-intubation when both provider and tool agree that UAO is present. There are low rates of re-intubation when the clinician labels the patient with UAO, but the tool does not. There are high rates of re-intubation when the tool detects UAO, but the clinician does

| | MD vs Tool | | RN vs Tool | | RT vs Tool | |
|---|---|---|---|---|---|---|
| | Re-intubation Rate | Total | Re-intubation (Rate) | Total | Re-intubation Rate | Total |
| **Both No UAO** | 15 (5.2%) | 290 | 11 (4%) | 276 | 13 (4.6%) | 283 |
| **Both Yes UAO** | 10 (27%) | 37 | 12 (29%) | 41 | 14 (30%) | 47 |
| **Clinician No Tool Yes** | 8 (20%) | 40 | 6 (17%) | 36 | 4 (13%) | 30 |
| **Clinician Yes tool No** | 1 (2.8%) | 36 | 5 (9%) | 56 | 3 (6%) | 49 |

*Risk Factors for post-extubation UAO*

Potential risk factors for all cause post-extubation UAO and subglottic UAO stratified by cuffed versus uncuffed endotracheal tubes are presented in Table 6-7.

Table 6: Risk factors for post extubation UAO and subglottic UAO for patients with cuffed ETTs. Data is presented at number (%) or Median (IQR). For P value calculations for subglottic vs. none, patients with supra glottic UAO were excluded from analysis. Significant p-values bolded. [1]Calculated as actual ETT size - ([Age(yrs)*0.25] +3.5 )

| Cuffed ETT | | | | | | |
|---|---|---|---|---|---|---|
| | No UAO 128 (76%) | UAO 41 (24%) | Subglottic UAO 21 (12%) | Total 169 (100%) | UAO vs None P value | Subglottic vs None P value |
| **Demographics** | | | | | | |
| Gender (M) | 73 (57%) | 24 (59%) | 14 (67%) | 97 (57%) | 0.87 | 0.36 |
| Genetic Abnormality | 19 (15%) | 8 (20%) | 5 (24%) | 27 (16%) | 0.48 | 0.3 |
| Neurologic Abnormality | 33 (26%) | 12 (29%) | 4 (19%) | 45 (27%) | 0.66 | 0.4 |
| Weight (kg) | 11.6 (5.5,28.4) | 11.75 (6.5,21.3) | 7.3 (5.4,11.1) | 11.1 (5.7,25.5) | 0.19 | **0.02** |
| Age (mo) | 20 (4,118) | 24 (5,91.5) | 7 (5,15) | 18 (5,108) | 0.48 | **0.05** |
| **Reason Intubation** | | | | | | |
| UAO | 5 (4%) | 11 (27%) | 6 (29%) | 16 (9%) | **0.0001** | **0.004** |
| Cardiac Surgery | 32 (25%) | 7 (17%) | 3 (17%) | 39 (23%) | 0.4 | 0.4 |
| Shock/CV | 13 (10%) | 6 (15%) | 3 (15%) | 19 (11%) | 0.6 | 0.8 |
| Parenchymal Lung | 21 (16%) | 5 (12%) | 4 (12%) | 26 (15%) | 0.7 | 0.9 |

(continued)

| Reason Intubation | | | | | | |
|---|---|---|---|---|---|---|
| Neurologic Abnormality | 20 (16%) | 6 (15%) | 2 (10%) | 26 (15%) | 0.9 | 0.9 |
| Extrathoracic | 23 (18%) | 3 (7%) | 1 (5%) | 26 (15%) | 0.9 | 0.1 |
| Lower Airway | 7 (5%) | 3 (7%) | 2 (10%) | 10 (6%) | 0.4 | 0.4 |
| Other | 7 (5%) | 0 (0%) | 0 (0%) | 7 (4%) | 0.13 | 0.3 |
| **Intubation Data** | | | | | | |
| ETT Size (mmID) | 4 (3.5,5.5) | 3.5 (3.5,4.5) | 3.5 (3.5,4) | 4 (3.5,5.5) | **0.05** | **0.002** |
| Actual ETT minus Cole[1] | -0.15 (-0.52,0.04) | -0.17 (-0.47,-0.02) | -0.1 (-0.44,0) | -0.17 (-0.52,-0.02) | 0.92 | 0.63 |
| Leak Intubation (n=96) | 64 (91%) | 18 (69%) | 9 (60%) | 82 (85%) | **0.006** | **0.002** |
| Leak Pressure Intubation (cm $H_2O$) (n=63) | 20 (15,25) | 20 (20,25) | 20.5 (20,25) | 20 (18,25) | **0.03** | **0.05** |
| Trauma Intubation (n=153) | 9 (7.5%) | 6 (17%) | 4 (23.5%) | 15 (10%) | 0.08 | **0.04** |
| >1 Intubation Attempt (n=132) | 18 (17.9%) | 9 (29%) | 6 (35.3%) | 27 (20%) | 0.17 | 0.1 |
| **Day of Extubation Data** | | | | | | |
| Cuff Inflated (Y) | 92 (73%) | 23 (56%) | 10 (47.6%) | 115 (69%) | **0.04** | **0.02** |
| Leak at Extubation (Y) | 93 (73%) | 25 (61%) | 9 (43%) | 118 (70%) | 0.15 | **0.006** |
| Leak Pressure Extubation (cm$H_2O$) | 20 (15,32) | 28 (20,40) | 35 (28,40) | 20 (18,35) | 0.12 | **0.0005** |
| Cuff Leak Volume (%) | 16.1 (0,34.8) | 5.5 (0,25) | 0 (0,6.3) | 0 (0,14) | 0.08 | **0.002** |
| GCS | 11 (9,11) | 11 (9,11) | 11 (10,11) | 11 (9,11) | 0.06 | 0.91 |
| Fluid Balance at midnight (cc/kg) | 11.5 (-5.7,28.7) | 7 (-7.8,22) | 8.2 (-6.5,22.1) | 9.3 (-6.8,28.3) | 0.44 | 0.4 |
| Pain Score | 1.3 (0.54,1.9) | 1.8 (0.8,2.6) | 2.1 (0.9,2.6) | 1.39 (0.67,2.1) | **0.04** | **0.02** |
| Length of Intubation | 124 (36.5,225) | 94 (50,170) | 119 (60,168) | 122 (40,214) | 0.54 | 0.88 |
| Steroids Pre Extubation | 14 (12.3%) | 10 (30%) | 5 (29.4%) | 24 (16%) | **0.01** | 0.06 |
| NIF | 40 (30,54) | 40 (30,55) | 40 (35,54) | 40 (30,55) | 0.54 | 0.63 |
| **Outcomes** | | | | | | |
| Re-intubated within 48Hrs | 4 (3%) | 7 (17%) | 5 (23.8%) | 11 (6.5%) | **0.002** | **0.0001** |
| Racemic Epi after Extubation | 6 (5%) | 23 (56%) | 16 (77%) | 29 (17%) | **0.0001** | **0.0001** |
| NIV after Extubation | 45 (35%) | 17 (41%) | 9 (43%) | 62 (37%) | 0.45 | 0.5 |
| Steroids after Extubation | 7 (5.5%) | 14 (34%) | 10 (48%) | 21 (12%) | **0.0001** | **0.0001** |

Table 7: Risk factors for post extubation UAO and subglottic UAO for patients with uncuffed ETTs. Data is presented at number (%) or Median (IQR). For P value calculations for subglottic vs. none, patients with supra glottic UAO were excluded from analysis. Significant p values bolded. [1] Calculated as actual ETT size - ([Age(yrs)*0.25] +4)

| | Uncuffed ETT | | | | | |
|---|---|---|---|---|---|---|
| | No UAO 174 (73%) | UAO 66 (27%) | Subglottic UAO 37 (15%) | Total 240 (100%) | UAO vs None P value | Subglottic vs None P value |
| **Demographics** | | | | | | |
| Gender (M) | 105 (60%) | 38 (58%) | 22 (59%) | 143 (60%) | 0.7 | 0.9 |
| Genetic Abnormality | 26 (15%) | 14 (21%) | 7 (19%) | 27 (16%) | 0.25 | 0.7 |
| Neurologic Abnormality | 13 (7%) | 7(11%) | 4 (11%) | 20 (8%) | 0.4 | 0.7 |
| Weight | 3.9 (3.2,6.2) | 4.1 (3.3, 6.4) | 4.1 (3.3,6.5) | 4 (3.2,6.2) | **0.02** | 0.08 |
| Age (mo) | 2 (0,6) | 2 (1,6) | 3 (1,6) | 2 (0,6) | **0.0002** | **0.001** |
| **Reason Intubation** | | | | | | |
| UAO | 13 (7%) | 18 (27%) | 8 (22%) | 31 (13%) | **0.0001** | **0.02** |
| Cardiac Surgery | 124 (71%) | 38 (58%) | 25 (67%) | 162 (68%) | 0.06 | 0.8 |
| Shock/CV | 14 (8%) | 6 (9%) | 1 (2.7%) | 20 (8%) | 1 | 0.42 |
| Parenchymal Lung | 8 (4.6%) | 0 (0%) | 0 (0%) | 8 (3%) | 0.08 | 0.2 |
| Neurologic Abnormality | 9 (5%) | 3 (5%) | 2 (5.4%) | 12 (5%) | 0.9 | 0.72 |
| Extrathoracic | 1 (1%) | 0 (0%) | 0 (0%) | 1 (0.5%) | 0.7 | 1 |
| Lower Airway | 4 (2.3%) | 1 (1.5%) | 1 (2.7%) | 5 (2%) | 0.9 | 1 |
| Other | 1 (1%) | 0 (0%) | 0 (0%) | 1 (0.5%) | 0.6 | 1 |
| **Intubation Data** | | | | | | |
| ETT Size (mmID) | 3.5 (3.5,4) | 3.5 (3.5,4) | 4 (3.5,4) | 3.5 (3.5,4) | 0.6 | 0.8 |
| Actual ETT minus Cole | -0.5 (-0.52,-0.1) | -0.5 (-0.54,-0.08) | -0.14 (-0.54,-0.06) | -0.5 (-0.54,-.1) | 0.12 | 0.4 |
| Leak Intubation (n=166) | 91 (78%) | 40 (80%) | 20 (72%) | 131 (78%) | 0.8 | 0.6 |
| Leak Pressure Intubation (cm $H_2O$) (n=118) | 20 (20,25) | 20 (20,25) | 20.5 (20,25) | 20.5 (20,25) | 0.4 | 1 |
| Trauma Intubation (n=219) | 6 (4%) | 6 (9%) | 3 (8%) | 12 (5%) | 0.1 | 0.5 |
| > 1 Intubation Attempt (n=199) | 17 (11.8%) | 10 (18%) | 4 (12.9%) | 27 (13.6%) | 0.24 | 0.5 |
| **Day of Extubation Data** | | | | | | |
| Leak at Extubation (Y) | 71 (41%) | 34 (50%) | 19 (51%) | 105 (44%) | 0.13 | 0.48 |
| Leak Pressure Extubation (cm$H_2O$) | 30 (20,40) | 29 (20,40) | 35 (20,40) | 30 (20,40) | 0.17 | 0.6 |
| Leak Percentage | 4.2 (0,19) | 6.5 (0.21.4) | 1.4 (0,6.2) | 4.2 (0,19) | 0.6 | 0.86 |

(continued)

| Day of Extubation Data | | | | | | |
|---|---|---|---|---|---|---|
| GCS | 11 (10,11) | 11 (10,11) | 11 (11,11) | 11 (10,11) | 0.09 | 0.06 |
| Fluid Balance at midnight (cc/kg) | 10 (-11.8,27) | 13.6 (-1.6,28.3) | 15 (-5,31.3) | 10.5 (-8.7,27.8) | **0.02** | **0.04** |
| Pain Score | 1.1 (0.56,1.8) | 1.8 (1.2,2.6) | 2.1 (1.4,2.6) | 1.4 (0.75,2.1) | 0.1 | **0.03** |
| Length of Intubation | 109 (35,200) | 122 (42,224) | 108 (50,213) | 109 (35,200) | 0.7 | 0.88 |
| Steroids Pre Extubation (n=193) | 15 (11%) | 10 (19%) | 4 (13%) | 25 (13%) | 0.1 | 0.4 |
| NIF | 38 (28,48) | 37 (28,46) | 35 (26,45) | 38 (28.5,48) | 0.55 | 0.9 |
| Outcomes | | | | | | |
| Re-intubated within 48Hrs | 10 (5.7%) | 13 (19.7%) | 10 (27%) | 23 (9.6%) | **0.001** | **0.0002** |
| Racemic Epi after Extubation | 11 (6%) | 42 (64%) | 31 (84%) | 53 (22%) | **0.0001** | **0.0001** |
| NIV after Extubation | 66 (38%) | 33 (50%) | 23 (62%) | 99 (41%) | 0.09 | **0.01** |
| Steroids after Extubation | 8 (4.6) | 23 (35%) | 16 (43%) | 31 (13%) | **0.0001** | **0.0001** |

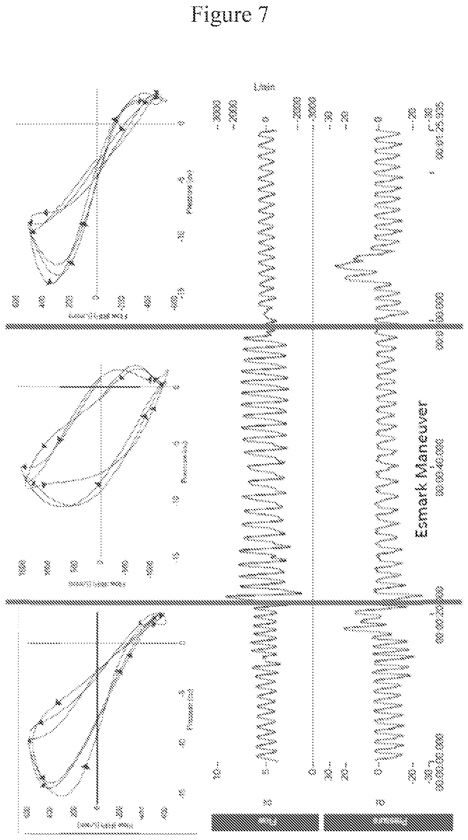

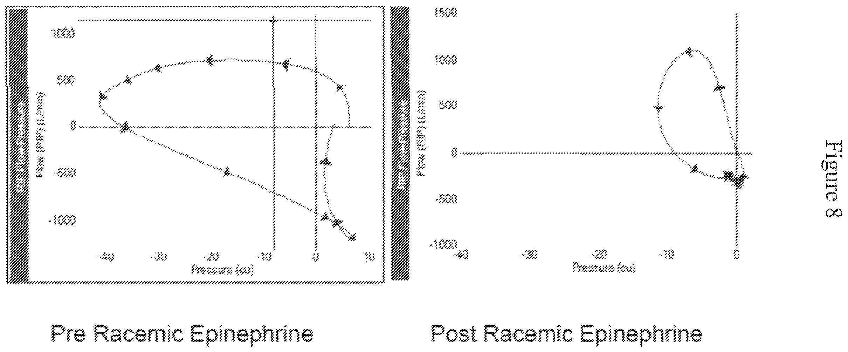

In addition, the outcome of UAO was split into all cases, and those which are likely not supraglottic. Figure 7 shows an example of supraglottic UAO, with a significant decrease in both flow limitation and pressure rate product during an Esmark Maneuver. Figure 8 shows an example of likely subglottic UAO, which had no improvement from an Esmark Maneuver, but a very significant response to racemic epinephrine.

*Age and cuffed vs. uncuffed ETT*

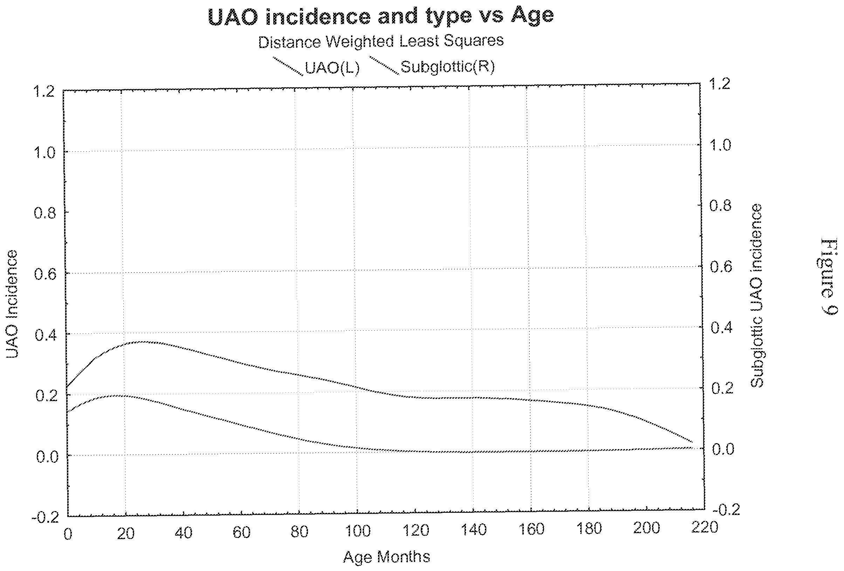

The incidence of all cause post-extubation UAO appears to peak near 2 years of age, with a slightly younger peak for those with likely subglottic UAO (Figure 9). Subglottic UAO is extremely uncommon for neonates (<1mo) and children > 5 years of age). Cuffed ETTs are rarely used in neonates (<1 mo), and uncuffed ETTs are rarely used for children > 18 months of age. There is no difference in the rate of post-extubation UAO or subglottic UAO between cuffed and uncuffed ETTs (Table 8). When stratifying by age, there is no difference in the rate of post-extubation UAO or subglottic UAO between cuffed and uncuffed ETTs for children < 18 months of age. There was a higher rate of post-extubation UAO for children with uncuffed ETT age 18mo-5 years than those with cuffed ETT (n=4 with uncuffed ETT in this age range) (Table 8).

Table 8: incidence of post-extubation UAO as a function of age and cuffed vs uncuffed ETT. Patient with uncuffed ETTs are younger. The highest rates of post extubation UAO and subglottic UAO occur in children 1mo-18 months of age. The rates of subglottic UAO are low for neonates (< 1mo) and children > 5 years. When stratifying by age, there is no difference in the incidence of UAO or subglottic UAO between cuffed and uncuffed ETTs.

| Age | All Patients | | | Cuffed ETT | | | Uncuffed ETT | | | Cuffed vs Uncuffed | |
|---|---|---|---|---|---|---|---|---|---|---|---|
| | UAO | Subglottic | Total | UAO | Subglottic | Total | UAO | Subglottic | Total | p value | P value |
| < 1mo | 5 7.7% | 1 1.5% | 65 15.8% | 0 0% | 0 0% | 3 1.7% | 5 8.1% | 1 1.6% | 62 25.8% | 0.61 | 0.83 |
| 1mo- 6mos | 53 28.6% | 34 18.4% | 185 45.2% | 11 21.1% | 9 17.3% | 52 31% | 42 31.6% | 25 18.8% | 133 55.5% | 0.16 | 0.81 |
| 6mo- 18mos | 26 42.1% | 18 28.6% | 63 15.4% | 14 47% | 9 30% | 30 17.8% | 12 36.4% | 9 27.2% | 33 13.8% | 0.41 | 0.81 |
| 18 mos- 5 years | 14 31.8% | 5 11.3% | 44 11.4% | 9 25% | 3 8.3% | 36 21.3% | 5 62.5% | 2 25% | 8 3% | 0.04 | 0.18 |
| > 5 years | 9 17.3% | 0 0% | 52 12.8% | 7 14.5% | 0 0% | 48 28.4% | 2 50% | 0 0% | 4 1.7% | 0.07 | NA |
| Total | 107 26.1% | 58 14.2% | 409 100% | 41 24.3% | 21 12.4% | 169 100% | 66 27.5% | 37 15.4% | 240 100% | 0.53 | 0.44 |

*Risk factors for those with cuffed ETT*

When examining children with cuffed endotracheal tubes (n=169; Table 6), there were 41 cases of post extubation UAO (23%). Risk factors include the absence of a leak at intubation, the cuff kept deflated on day of extubation, higher average 24 hour pain scores, use of steroids prior to extubation, and being intubated primarily for UAO. There were 21 cases (12%) of UAO which were likely not supraglottic. All the above risk factors were associated with subglottic UAO, in addition to the presence of a leak at extubation, leak pressure at extubation, cuff leak volume at extubation, and trauma at intubation. A cut-off value of cuff leak volume < 10%, or leak pressure (with cuff deflated) > 25 cmH$_2$0 yielded positive likelihood ratios between 2-2.5, with negative likelihood ratios of 0.2 on the outcome of subglottic UAO (Table 9). If the estimated prevalence of subglottic UAO is 15% (as it is in our cohort); these test characteristics result in reassuring post-test probabilities when a leak is present (approximately 4%). When a leak is absent at the above pressure, the posttest probability of subglottic UAO ranges from 28-31%. In a multivariate model, cuff leak volume or extubation leak pressure, average pain score, and pre-existing UAO remained associated with subglottic post extubation UAO (all p<0.04). The model had good discrimination ability for subglottic UAO (AUC 0.82).

Table 9: Extubation leak pressure and cuff leak volume for cuffed ETTs. A leak pressure of 25 cmH$_2$0 with the cuff deflated or a cuff leak volume of 10% yields good test statistics to identify low and high risk patients for post extubation UAO.

| Cuffed ETT | | | | | | | | | | |
|---|---|---|---|---|---|---|---|---|---|---|
| **Statistic** | **Leak Pressure (cmH$_2$0)** | | | | | **Leak Percentage (%)** | | | | |
| Leak | >20 | >25 | >30 | >35 | >40 | 0 | <5 | <10 | <15 | <20 |
| Total | 123 | 82 | 65 | 44 | 37 | 52 | 64 | 77 | 88 | 105 |
| No UAO | 103 | 64 | 50 | 33 | 27 | 37 | 50 | 60 | 71 | 86 |
| Subglottic | 20 | 18 | 15 | 11 | 10 | 11 | 14 | 17 | 17 | 19 |
| Fraction Having Subglottic UAO | 0.163 | 0.220 | 0.231 | 0.250 | 0.270 | 0.212 | 0.219 | 0.221 | 0.193 | 0.181 |
| Sensitivity | 0.952 | 0.857 | 0.714 | 0.524 | 0.476 | 0.524 | 0.667 | 0.810 | 0.810 | 0.905 |
| Specificity | 0.304 | 0.568 | 0.662 | 0.777 | 0.818 | 0.723 | 0.662 | 0.595 | 0.520 | 0.419 |
| Positive LR | 1.368 | 1.982 | 2.115 | 2.349 | 2.611 | 1.892 | 1.973 | 1.997 | 1.688 | 1.557 |
| Negative LR | 0.158 | 0.252 | 0.431 | 0.613 | 0.641 | 0.658 | 0.503 | 0.320 | 0.366 | 0.227 |
| Pretest Probability | 15% | 15% | 15% | 15% | 15% | 15% | 15% | 15% | 15% | 15% |
| Post Test Probability (w/ Leak) | 3% | 4% | 8% | 10% | 11% | 12% | 9% | 6% | 7% | 4% |
| Post Test Probability (w/o Leak) | 24% | 35% | 37% | 42% | 46% | 33% | 35% | 35% | 30% | 28% |
| ROC | 0.628 | 0.713 | 0.688 | 0.65 | 0.647 | 0.622 | 0.664 | 0.702 | 0.665 | 0.662 |

*Risk Factors for those with uncuffed ETT*

For children with uncuffed endotracheal tubes (n=240, Table 7), there were 66 cases of post-extubation UAO (32%). Risk factors included pre-existing history of UAO, age 1month to 5 years, and higher fluid balance at midnight before extubation. There were 37 cases of UAO which were likely not supraglottic (15%), and all the above risk factors remained associated with subglottic UAO, except pre-existing UAO. Higher average pain scores in the 24 hours prior to extubation was also associated with subglottic/ UAO. No leak pressure values or percentages were associated with post-extubation UAO with positive and negative likelihood ratios all very close to 1 (Table 10). In a multivariate model only age 1mo-5 years remained associated with subglottic post extubation UAO, with only fair discrimination ability for subglottic UAO (AUC 0.68).

Table 10: For uncuffed ETTs, there is no extubation leak pressure or leak percentage which is helpful to differentiate low from high risk patients.

| Uncuffed ETT | | | | | | | | | | |
|---|---|---|---|---|---|---|---|---|---|---|
| **Statistic** | **Leak Pressure (cmH$_2$0)** | | | | | **Leak Percentage (%)** | | | | |
| Leak | >20 | >25 | >30 | >35 | >40 | 0 | <5 | <10 | <15 | <20 |
| Total | 217 | 188 | 164 | 131 | 112 | 135 | 159 | 185 | 202 | 216 |
| No UAO | 185 | 161 | 139 | 109 | 95 | 115 | 132 | 155 | 172 | 185 |
| Subglottic | 32 | 27 | 25 | 22 | 17 | 20 | 27 | 30 | 30 | 31 |
| Fraction Having Subglottic UAO | 0.147 | 0.144 | 0.152 | 0.168 | 0.152 | 0.148 | 0.170 | 0.162 | 0.149 | 0.14 |
| Sensitivity | 0.865 | 0.730 | 0.676 | 0.595 | 0.460 | 0.540 | 0.730 | 0.811 | 0.811 | 0.83 |
| Specificity | 0.084 | 0.203 | 0.312 | 0.460 | 0.530 | 0.428 | 0.343 | 0.229 | 0.144 | 0.08 |
| Positive LR | 0.944 | 0.916 | 0.982 | 1.102 | 0.977 | 0.944 | 1.111 | 1.051 | 0.948 | 0.91 |
| Negative LR | 1.605 | 1.332 | 1.040 | 0.881 | 1.020 | 1.075 | 0.787 | 0.827 | 1.311 | 2.03 |
| Pretest Probability | 15% | 15% | 15% | 15% | 15% | 15% | 15% | 15% | 15% | 15% |
| Post Test Probability (w/ Leak) | 28% | 24% | 18% | 16% | 18% | 19% | 14% | 15% | 23% | 36% |
| Post Test Probability (w/o Leak) | 17% | 16% | 17% | 19% | 17% | 17% | 20% | 19% | 17% | 16% |
| ROC | 0.475 | 0.466 | 0.494 | 0.528 | 0.495 | 0.4842 | 0.537 | 0.520 | 0.478 | 0.45 |

**Example 5**

We have demonstrated that an objective parameter used to measure inspiratory flow limitation and effort of breathing as a marker of post extubation UAO 5 minutes after extubation is highly predictive of re-intubation. Using this marker can help reduce some of the variability inherent to clinical assessment of UAO or stridor scoring systems, as it appears to perform better than bedside clinical assessment at stratifying re-intubation risk. Moreover, using this objective parameter has allowed us to identify risk factors for the development of post extubation UAO, which appear to be different based on age and the presence of cuffed versus uncuffed endotracheal tubes.

Overall, the rates of post-extubation UAO are comparable between children with cuffed (24.3%) and uncuffed endotracheal tubes (27.5%). However, it appears as if leak pressures (both on intubation and extubation) and leak percentages are only relevant for cuffed endotracheal tubes. To that end, if a patient has a cuffed endotracheal tube and has a low cuff leak volume at extubation (<10%), they are 2.5 times more likely to develop subglottic UAO after extubation than a patient with a cuff leak volume > 10%. Similar results can be observed for leak pressure with the cuff deflated for cuffed endotracheal tubes, using a cut-off pressure of <25 cmH$_2$0. Children with cuffed endotracheal tubes who did not have a leak on intubation were nearly 6 times more likely to have subglottic post extubation UAO than those who had a leak (Table 6). For uncuffed endotracheal tubes, no leak pressure or percentage was relevant at either intubation or extubation, although there is substantial missing data on intubation leak. Therefore, the common practice of delaying extubation to administer corticosteroids to a patient who has an uncuffed endotracheal tube and does not have a leak at extubation cannot be justified by our data. A possible explanation for this difference between cuffed and uncuffed endotracheal tubes may relate to the fact that a relatively smaller tube is used when it is cuffed because the cuff can be inflated to maintain tidal volume and pressure. Because the cuff sits in the subglottic space, the cuff leak volume percentage is more reflective of subglottic edema. From the leak at intubation data, it is clear that if there is no leak at intubation with a cuffed tube, the patient is high risk for post-extubation UAO. Hence, careful attention should likely be given to intubation leak, particularly with a cuffed tube. If a leak is large with an uncuffed tube, it is often changed, particularly if it is compromising ventilation. Perhaps this is why leak pressures are less predictive for uncuffed tubes, because by necessity minimal leak is needed for many children.

Other risk factors for post extubation UAO regardless of type of tube include age, higher pain scores, and a history of pre-existing UAO. Many of these patients were post op airway surgery, or were intubated primarily for upper airway obstruction (i.e. croup). The age groups identified in this study as highest risk for subglottic UAO are children between 1 month and 2 years of age (32% incidence UAO, 19.2% subglottic) and children 2-5 years of age (35% incidence UAO, 14.2% subglottic), although the later was only 8% of the population and is underpowered. This higher risk may be related to choice of size of endotracheal tube (many potential choices to use from 3.5-5.5 based on interpretation of formulae), developmental issues related to the trachea in this age group, or sedation. We found pain scores (as a surrogate for level of sedation in the 24 hours prior to extubation) to be associated with post-extubation UAO, and children in this age range are typically amongst the most difficult the keep calm. However in the multivariate models controlling for age, pain scores were no longer significant. This importance of sedation consistent with results from the recent RESTORE clinical trial, which found a higher incidence of clinically judged post extubation stridor in the group of patients who were, in general, less sedated. The pain scores we used in our study are an imprecise and very crude surrogate for sedation, but reinforce that level of sedation or agitation may be relevant for the development of post-extubation UAO, particularly for children between 1 month and 5 years of age. Neonates < 1month have a very low risk of post-extubation UAO. There are several possible explanations for this finding, perhaps related to the relative elasticity of the neonatal trachea making it less subject to trauma or inflammation. It may also be an issue of endotracheal tube sizing, and less variability in size of ETT chosen for a child < 1 month of age, although our analysis of the actual ETT size minus the size predicted from the cole formula (Table 6-7) would not support this explanation. While 14% of children > 5 have post-extubation UAO, all of these cases were likely supraglottic.

In addition to using the objective parameter of UAO severity to identify risk factors for post-extubation UAO, if implemented in real time, such a parameter may be helpful to guide therapy. The high predictive ability it provides 5 minutes after extubation of re-intubation may help identify a group of patients that warrant immediate treatment, assuming the UAO can be modified with other therapies such as racemic epinephrine, heliox, corticosteroids, etc.

The inventors have demonstrated that a physiology based objective marker of post extubation UAO is highly predictive of re-intubation when used 5 minutes after extubation, and better than clinical assessment. Risk factors for post-extubation UAO include age 1mo-5 years, poor sedation, pre-existing UAO, and if a cuffed endotracheal tube is used, then cuff leak volume or leak pressures. Leak pressures do not aid clinical decision-making for uncuffed endotracheal tubes.

## Example 6

*Persistent elevation in effort or work of breathing from persistent respiratory disease*

In addition to upper airway obstruction resulting in persistent elevation in effort or work of breathing and extubation failure, persistent respiratory diseases may also result in extubation failure. To assess persistent elevations in effort of breathing, the systems described herein can identify patients who have higher than expected effort of breathing either before extubation, or immediately after extubation. Data from 409 patients demonstrates that in particular the Pressure Rate Product (PRP), which we use to evaluate effort of breathing, is predictive of extubation failure. We obtained standardized assessments of effort of breathing while spontaneously breathing on Pressure Support of 10/5 cmH20, and Continuous Positive Airway Pressure (CPAP) of 5 prior to extubation, as well as 5 and 60 minutes after extubation. Of the 409 patients included in the analysis, 8% required re-intubation within 48 hours of extubation. Over half of these re-intubations were in the setting of UAO (measured objectively using the combination of esophageal manometry and RIP, see above), and 40% of patients were placed on non-invasive ventilation after extubation (High-Flow Nasal Cannula, Nasal intermittent Mandatory Ventilation, CPAP, or BiPAP). When excluding patients with UAO after extubation (N=107) PRP on CPAP or PS was significantly higher for patients who were re-intubated or placed on NIV after extubation (p<0.001), and had fair ability to discriminate patients likely to fail extubation (AUC 0.64 (0.58, 0.69). The pressure rate product 5 minutes after extubation was even more predictive at identifying patients at high risk of being reintubated or receiving NIV (p=0.0002, AUC 0.694 (0.64,0.75). Although only 14 patients were re-intubated from causes other than UAO, effort of breathing values just after extubation (p=0.05), as well as the change in this value from CPAP (p= 0.1), was predictive of re-intubation. These values appear more predictive than current evidence based methods (Rapid Shallow Breathing Index, spontaneous tidal volume, p>0.2).

**Example 7**

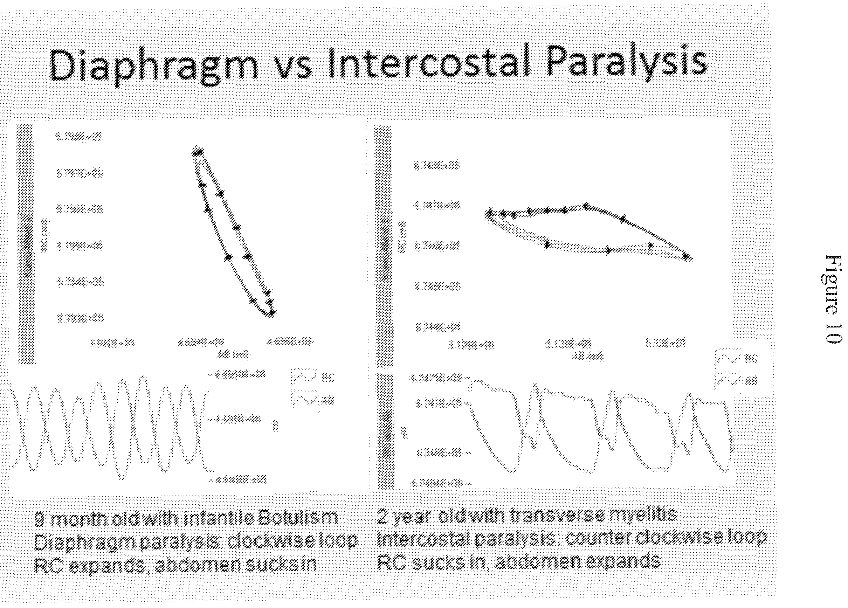

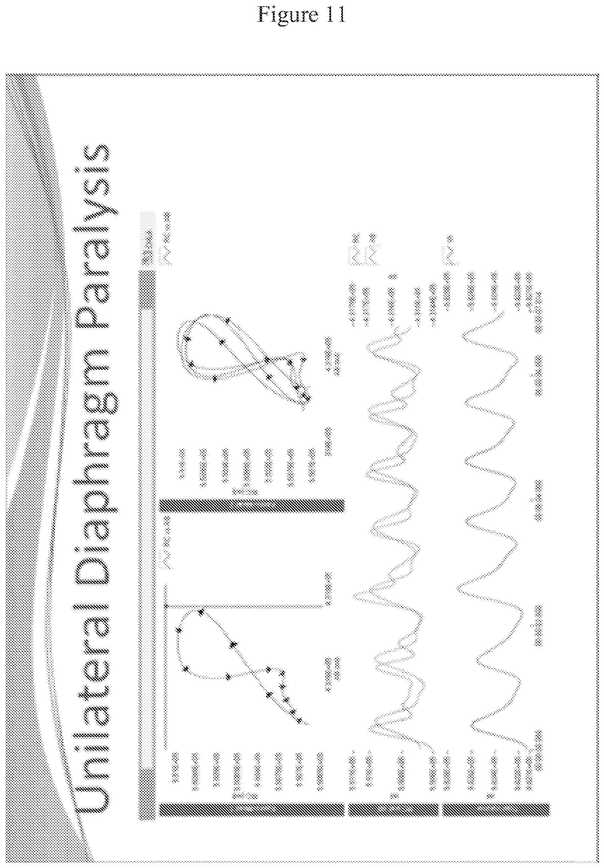

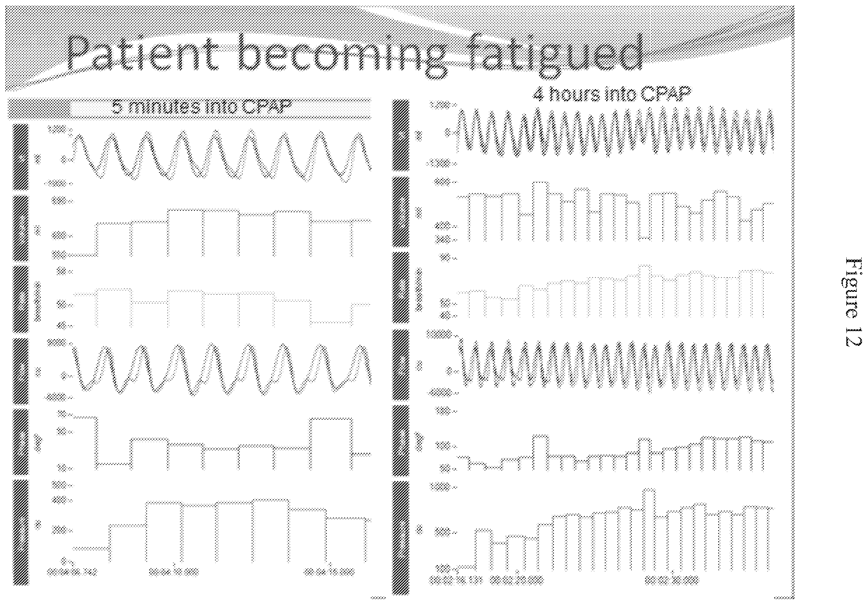

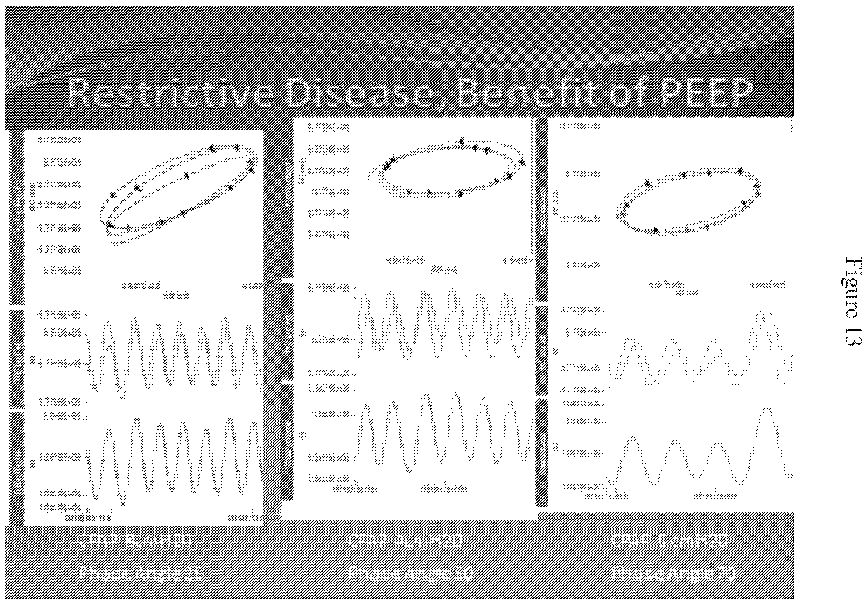

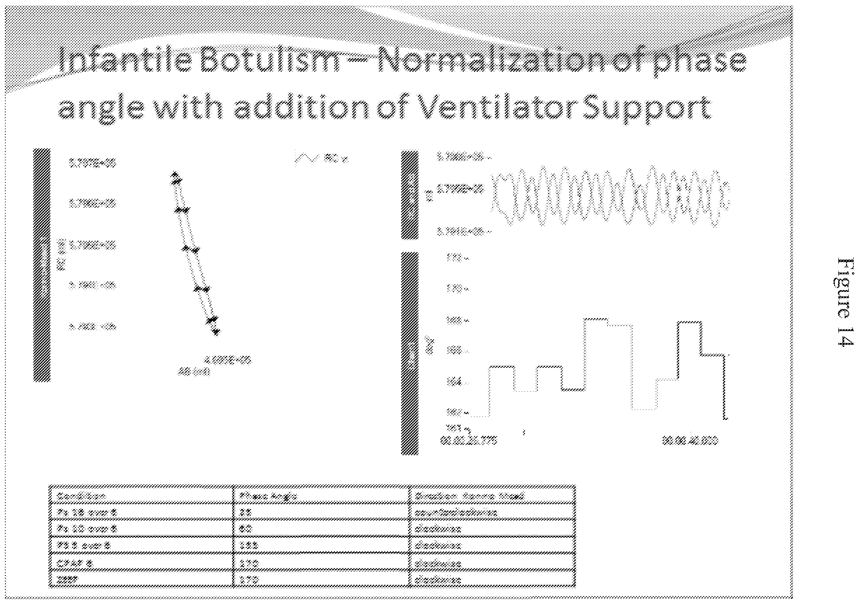

In addition to upper airway obstruction causing extubation failure, neuromuscular weakness may also result in extubation failure. The methods described herein can identify patients with neuromuscular weakness, originating from the intercostal muscles (Figure 10), or unilateral or bilateral diaphragms (Figure 11). This is done by examining the direction of Konno-Mead Plots and the phase angle. It can identify patients who are becoming fatigued on spontaneous breathing trials and are likely to fail extubation (Figure 12) by combining information from RIP and PRP. It can identify optimal levels of ventilator support to normalize respiratory mechanics for patients with a variety of disease conditions including restrictive lung disease or neuromuscular weakness (Figure 13 and 14).

**Example 8**

*Role of synchrony in non-invasive mechanical ventilation in infants*

For infants, clinicians frequently use nasal modes of non-invasive ventilation including high flow nasal cannula (HFNC) and nasal intermittent mechanical ventilation (NIMV). Frequently, practitioners pay little attention to patient-device synchrony for NIMV, but synchrony may have large effects on effort of breathing. We sought to directly compare effort of breathing between HFNC and NIMV, stratified by degree of NIMV synchrony.

We conducted a prospective randomized cross-over trial for patients < 6 months of age admitted to the cardiothoracic or PICU. Children were eligible if they received non-invasive ventilation after extubation. We measured effort of breathing using esophageal manometry with pressure rate product (PRP = peak-to-trough change in esophageal pressure * respiratory rate) under the following conditions: intubated on CPAP 5 $cmH_2O$ (CPAP5), HFNC of 6 lpm (HFNC6), NIMV R20, PEEP 5 $cmH_2O$ with IPAP of 12 (NIMV12), and the same NIMV settings using the Neoseal nasal clip (Neo12). Primary outcome was the difference in effort of breathing using PRP between HFNC and NIMV with and without Neoseal. Secondary outcomes included the effect of NIMV synchrony on effort of breathing, as well as dose response effects of HFNC and NIMV. Synchrony was determined by comparing patient breaths (esophageal manometry) with mechanically delivered breaths (spirometry in ventilator circuit) over a one minute period. HFNC was obtained using the Fisher and Paykel system, and NIMV used the Maquet systems on the Servo-I ventilator. We used RAM cannulae as the nasal interface for both arms. For a subset of patients, we performed NIMV measurements both with and without a nasal clip (NeoSeal) to see if this could improve synchrony.

Measurements while on non-invasive ventilation were performed 5 minutes - 2 hours after extubation. Synchrony was determined comparing patient initiated breaths (esophageal manometry) to ventilator delivered breaths (ventilator spirometry). A percentage synchrony parameter was calculated which quantified the number of breaths in a 2 minute period in which patient effort (as guaged by esophageal manometry deflections), resulted in a delivered breath from the non-invasive ventilator (as guaged by flow seen when the spirometer was put in line with the NIV circuit). The % synchrony was calculated as the (# of spirometry breaths with an esophageal pressure trigger)/ total number of patient efforts (all esophageal pressure triggers). The primary analysis compared effort of breathing on HFNC of 6 lpm to NIMV of IPAP12/EPAP 5. Secondary analysis included flow rate or NIMV IPAP on effort of breathing, NIMV synchrony on effort of breathing, whether the NeoSeal improved synchrony, and whether the NeoSeal reduced effort of breathing.

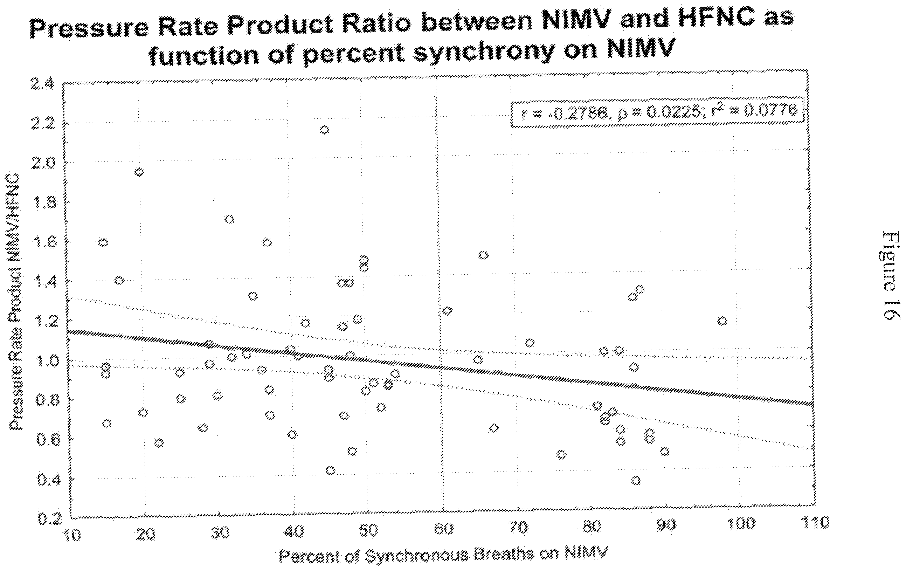

Forty-two children were included. Median (IQR) age was 2 (0.5, 4) months. The mean PRP while intubated on CPAP5 297 ($\pm$152) was lower than HFNC6 403 ($\pm$183), NIMV12 382 ($\pm$199) and Neo12 360 ($\pm$ 148). There was no difference in mean PRP between HFNC6 and NIMV12 (p = 0.36), or NIMV12 and Neo12 (p= 0.5). However, PRP on Neo12 was lower than on HFNC6 (p= 0.04). When patients were crossed-over to receive Neoseal while on NIMV (n=26), synchrony improved slightly (47% to 58%). Patients who achieved more synchrony (regardless of Neoseal) had a larger reduction in effort of breathing on NIMV compared to HFNC (R=-0.28, p=0.022, Figure 16). When the percentage of synchronized breaths exceeded 60%, NIMV resulted in a statistically significant reduction in effort of breathing compared to HFNC (p=0.03).

Table 11: Demographics of the 42 enrolled patients are summarized as median (range) or number (percent).

| Patient Demographics (N = 42) | |
|---|---|
| Age (mo) | 2 (0.5-4) |
| Weight (kg) | 3.6 (2.2-9.6) |
| Sex (male) | 22(52%) |
| Post cardiac surgery | 30(71%) |
| Ventilator Days (days) | 7 days (2-30) |

(continued)

| Patient Demographics (N = 42) | |
|---|---|
| Non-invasive Ventilator Days (days) | 1 day (0.2-29) |
| Number receiving NeoSeal nasal clip | 26 (62%) |

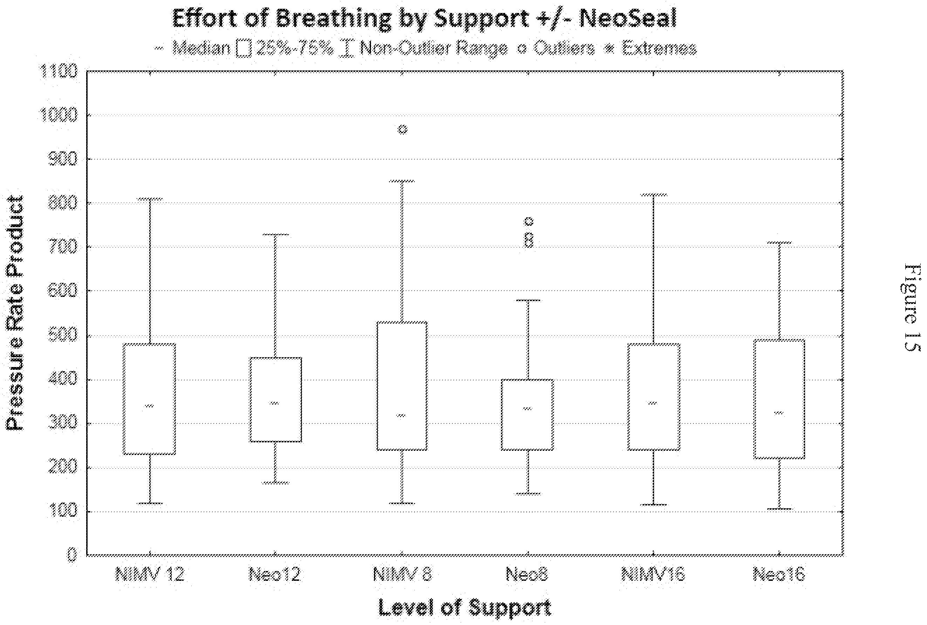

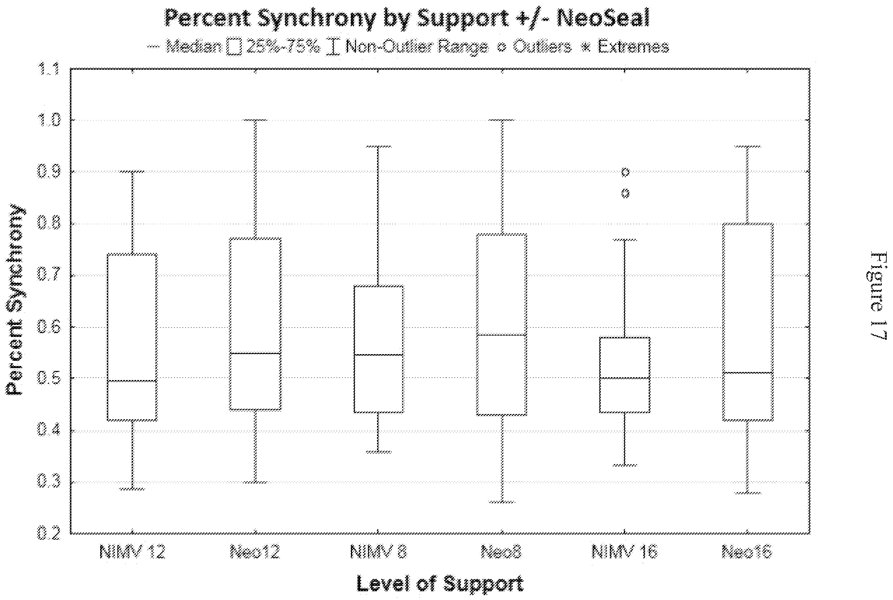

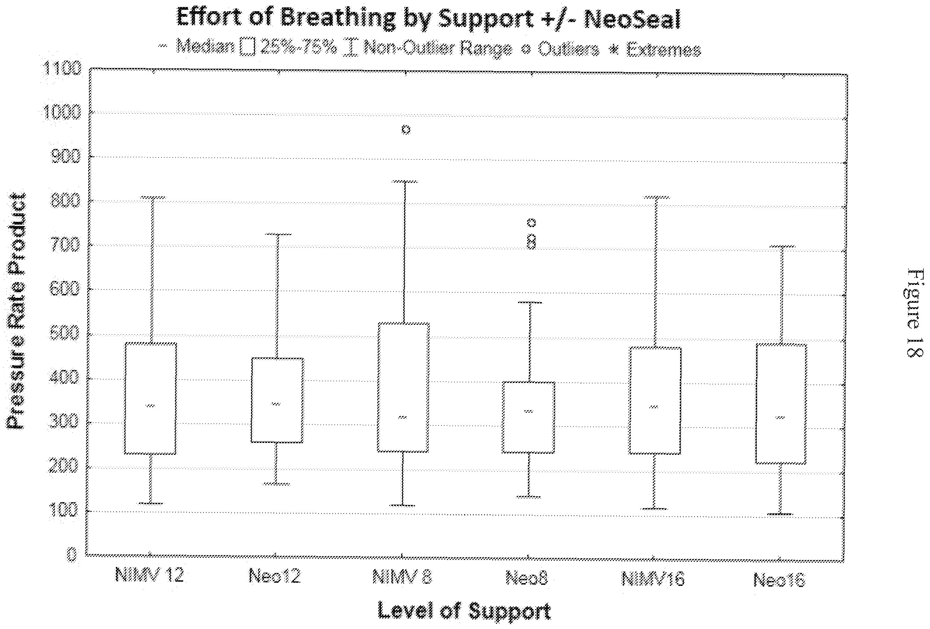

As shown in Figure 15, the primary analysis demonstrated effort of breathing before extubation on CPAP was lower than all other conditions (p = .002). However, there is no difference in effort of breathing between HFNC and NIMV, regardless of flow rate or inspiratory pressure (KW ANOVA p= 0.15). Further, as shown in Figure 16, secondary analysis demonstrated patients who achieved more synchrony had a larger reduction in effort of breathing on NIMV compared to HFNC (NIMV12 vs HFNC6 and Neo12 vs HFNC6). (R= -0.28, p=0.022, $r^2$ = 0.0778). NIMV results in lower effort of breathing than HFNC when synchrony exceeds 60% (Green line). As shown in Figure 17, in the 26 patients who had the addition of NeoSeal, synchrony improved from 50% to 55% (p = 0.04). As shown in Figure 18, in the 26 patients who had the addition of NeoSeal, synchrony improved from 50% to 55% (p = 0.04).

For infants on NIMV, effort of breathing is reduced as patient/mechanical ventilator synchrony improves, and NIMV may be superior to HFNC when NIMV synchrony exceeds 60%.

The various methods and techniques described above provide a number of ways to carry out the application. Of course, it is to be understood that not necessarily all objectives or advantages described can be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as taught or suggested herein. A variety of alternatives are mentioned herein. It is to be understood that some preferred embodiments specifically include one, another, or several features, while others specifically exclude one, another, or several features, while still others mitigate a particular feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be employed in various combinations by one of ordinary skill in this art to perform methods in accordance with the principles described herein. Among the various elements, features, and steps some will be specifically included and others specifically excluded in diverse embodiments.

Although the application has been disclosed in the context of certain embodiments and examples, it will be understood by those skilled in the art that the embodiments of the application extend beyond the specifically disclosed embodiments to other alternative embodiments and/or uses and modifications and equivalents thereof.

Preferred embodiments of this application are described herein, including the best mode known to the inventors for carrying out the application. Variations on those preferred embodiments will become apparent to those of ordinary skill in the art upon reading the foregoing description. It is contemplated that skilled artisans can employ such variations as appropriate, and the application can be practiced otherwise than specifically described herein. Accordingly, many embodiments of this application include all modifications and equivalents of the subject matter recited in the claims appended hereto as permitted by applicable law. Moreover, any combination of the above-described elements in all possible variations thereof is encompassed by the application unless otherwise indicated herein or otherwise clearly contradicted by context.

All patents, patent applications, publications of patent applications, and other material, such as articles, books, specifications, publications, documents, things, and/or the like, referenced herein are hereby incorporated herein by this reference in their entirety for all purposes, excepting any prosecution file history associated with same, any of same that is inconsistent with or in conflict with the present document, or any of same that may have a limiting affect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

It is to be understood that the embodiments of the application disclosed herein are illustrative of the principles of the embodiments of the application. Other modifications that can be employed can be within the scope of the application. Thus, by way of example, but not of limitation, alternative configurations of the embodiments of the application can be utilized in accordance with the teachings herein. Accordingly, embodiments of the present application are not limited to that precisely as shown and described.

Various embodiments of the invention are described above in the Detailed Description. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s).

The foregoing description of various embodiments of the invention known to the applicant at this time of filing the application has been presented and is intended for the purposes of illustration and description. The present description is not intended to be exhaustive nor limit the invention to the precise form disclosed and many modifications and variations are possible in the light of the above teachings. The embodiments described serve to explain the principles of the invention and its practical application and to enable others skilled in the art to utilize the invention in various embodiments and with various modifications as are suited to the particular use contemplated. Therefore, it is intended that the invention not be limited to the particular embodiments disclosed for carrying out the invention.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the scope of this invention.

**Claims**

1. A method of operating a computer system for evaluating medical conditions relating to effort of breathing in a subject in need thereof, the computer system comprising one or more processors, a display and memory storing one or more programs comprising instructions that when executed by the one or more processors cause the processors to:

   obtain, from a first sensor, first biometric signals wherein the first biometric signals are respiratory inductance plethysmography (RIP) and obtain, from a second sensor, second biometric signals, wherein the second biometric signals are esophageal pressure (Pes);
   calibrate the RIP signals, wherein the RIP signals are calibrated so as to provide a signal proportional to a tidal volume;
   determine, from the first and second biometric signals a relative effort of breathing value and plot the calibrated RIP signals against the Pes signals to obtain a flow pressure loop; and
   display on the display a report comprising an interpretation of the effort of breathing value, wherein the interpretation is updated as additional data relating to the flow pressure loops is received.

2. The method of claim 1, wherein the RIP signal is calibrated while the subject is intubated, prior to extubation, so as to allow for RIP calibration in real-time; or wherein the RIP signal is calibrated using a method selected from the group consisting of continuous positive airway pressure, a quantitative diagnostic calibration (QDC), and negative inspiratory force (NIF).

3. The method of claim 1, wherein the PRP is obtained over a pre-determined amount of time of breathing by the subject comprising any one or more of 10 seconds, 20 seconds, 30 seconds, 40 seconds,50 seconds, 60 seconds, 90 seconds, 120 seconds, 150 seconds, 180 seconds, 240 seconds, 300 seconds, 360 seconds, or a combination thereof; or wherein PRP is obtained by identifying the peak and trough change of the esophageal pressure signals for each breath and multiplying by the respiratory rate.

4. A method of claim 1, wherein the medical conditions relating to effort of breathing is the likelihood of extubation failure.

5. A non-transitory computer-readable storage medium storing one or more programs for evaluating medical conditions relating to effort of breathing in a subject in need thereof, the one or more programs for execution by one or more processors of a computer system, the one or more programs comprising instructions for:

   obtaining from a first sensor first biometric signals, wherein the first biometric signals are respiratory inductance plethysmography (RIP), and obtaining from a second sensor second biometric signals, wherein the second biometric signals are esophageal pressure (Pes);
   calibrating the RIP signals, wherein the RIP signals are calibrated so as to provide a signal proportional to a tidal volume;
   determining, from the first and second biometric signals, a relative effort of breathing value and plotting the calibrated RIP signals against the Pes signals to obtain a flow pressure loop;
   displaying a report comprising an interpretation of the effort of breathing value, wherein the interpretation is updated as additional data relating to the flow pressure loops is received.

**Patentansprüche**

1. Ein Verfahren zum Betreiben eines Computersystems zur Auswertung von medizinischen Zuständen, die sich auf die Atemanstrengung eines Subjekts bezieht, die dies benötigt, das Computersystem umfassend einen oder mehrere Prozessoren, eine Anzeige und einen Speicher, der eines oder mehrere Programme speichert, die Befehle umfassen, welche, wenn sie von dem einem oder mehreren Prozessoren ausgeführt werden, die Prozessoren dazu veranlassen:

   erhalten erster biometrischer Signale von einem ersten Sensor, wobei die ersten biometrischen Signale respiratorische Induktionsplethysmographie (RIP) sind, und erhalten zweiter biometrischer Signale von einem zweiten Sensor, wobei die zweiten biometrischen Signale Ösophagusdruck (Pes) sind;
   kalibrieren der RIP-Signale, wobei die RIP-Signale so kalibriert werden, dass sie ein Signal proportional zu einem Tidalvolumen liefern;
   bestimmen eines Werts aus den ersten und zweiten biometrischen Signalen für die relative Atemanstrengung und aufzeichnen der kalibrierten RIP-Signale gegen die Pes-Signale, um eine Fluss-Druck-Schleife zu erhalten; und
   anzeigen eines Berichts auf der Anzeige, der eine Interpretation des Wertes der Atemanstrengung umfasst, wobei die Interpretation aktualisiert wird, wenn zusätzliche Daten in Bezug auf die Fluss-Druck-Schleifen empfangen werden.

2. Das Verfahren gemäß Anspruch 1, wobei das RIP-Signal kalibriert wird, während das Subjekt vor der Extubation intubiert ist, um eine RIP-Kalibrierung in Echtzeit zu ermöglichen; oder wobei das RIP-Signal unter Verwendung eines Verfahrens kalibriert wird, das aus der Gruppe bestehend aus kontinuierlichem positiven Atemwegsdruck, einer quantitativen diagnostischen Kalibrierung (QDC) und negativer Inspirationskraft (NIF) ausgewählt wird.

3. Das Verfahren gemäß Anspruch 1, wobei das PRP über eine vorbestimmte Zeitspanne der Atmung des Subjekts erhalten wird, die eines oder mehr von 10 Sekunden, 20 Sekunden, 30 Sekunden, 40 Sekunden, 50 Sekunden, 60 Sekunden, 90 Sekunden, 120 Sekunden, 150 Sekunden, 180 Sekunden, 240 Sekunden, 300 Sekunden, 360 Sekunden oder eine Kombination davon umfasst; oder wobei das PRP durch Identifizierung der Spitzen- und Tiefveränderung der Ösophagusdrucksignale für jeden Atemzug und Multiplikation mit der Atmungsrate erhalten wird.

4. Ein Verfahren gemäß Anspruch 1, wobei die medizinischen Zustände, die sich auf die Atemanstrengung beziehen, die Wahrscheinlichkeit eines Extubationsversagens ist.

5. Ein nicht-transitorisches, computerlesbares Speichermedium, das ein oder mehrere Programme zur Bewertung medizinischer Zustände in Bezug auf die Atemanstrengung eines Subjekts speichert, wobei das eine oder die mehreren Programme zur Ausführung durch einen oder mehrere Prozessoren eines Computersystems, wobei das eine oder die mehreren Programme Anweisungen umfassen zum:

   erhalten erster biometrischer Signale von einem ersten Sensor, wobei die ersten biometrischen Signale respiratorische Induktionsplethysmographie (RIP) sind, und erhalten zweiter biometrischer Signale von einem zweiten Sensor, wobei die zweiten biometrischen Signale Ösophagusdruck (Pes) sind;
   kalibrieren der RIP-Signale, wobei die RIP-Signale so kalibriert werden, dass sie ein Signal proportional zu einem Tidalvolumen liefern;
   bestimmen eines Werts aus den ersten und zweiten biometrischen Signalen für die relative Atemanstrengung und aufzeichnen der kalibrierten RIP-Signale gegen die Pes-Signale, um eine Fluss-Druck-Schleife zu erhalten; und
   anzeigen eines Berichts auf der Anzeige, der eine Interpretation des Wertes der Atemanstrengung umfasst, wobei die Interpretation aktualisiert wird, wenn zusätzliche Daten in Bezug auf die Fluss-Druck-Schleifen empfangen werden.

**Revendications**

1. - Procédé de fonctionnement d'un système informatique pour évaluer des conditions médicales relatives à un effort de respiration chez un sujet qui en a besoin, le système informatique comprenant un ou plusieurs processeurs, un dispositif d'affichage et une mémoire stockant un ou plusieurs programmes comprenant des instructions qui, lorsqu'elles sont exécutées par le ou les processeurs, amènent les processeurs à :

obtenir, à partir d'un premier capteur, des premiers signaux biométriques, les premiers signaux biométriques étant une pléthysmographie respiratoire par inductance (RIP) et obtenir, à partir d'un second capteur, des seconds signaux biométriques, les seconds signaux biométriques étant une pression oesophagienne (Pes) ; étalonner les signaux de RIP, les signaux de RIP étant étalonnés de façon à fournir un signal proportionnel à un volume courant ;

déterminer, à partir des premiers et seconds signaux biométriques, une valeur d'effort de respiration relative et tracer les signaux de RIP étalonnés contre les signaux de Pes pour obtenir une boucle de pression d'écoulement ; et

afficher, sur le dispositif d'affichage, un rapport comprenant une interprétation de la valeur d'effort de respiration, l'interprétation étant mise à jour au fur et à mesure que des données supplémentaires relatives aux boucles de pression d'écoulement sont reçues.

2. - Procédé selon la revendication 1, dans lequel le signal de RIP est étalonné pendant que le sujet est intubé, avant l'extubation, de façon à permettre un étalonnage de RIP en temps réel ; ou dans lequel le signal de RIP est étalonné en utilisant un procédé choisi parmi le groupe consistant en une pression positive expiratoire continue, un étalonnage de diagnostic quantitatif (QDC) et une force inspiratoire négative (NIF).

3. - Procédé selon la revendication 1, dans lequel le produit pression-fréquence (PRP) est obtenu sur une durée prédéterminée de respiration du sujet comprenant une ou plusieurs durées parmi 10 secondes, 20 secondes, 30 secondes, 40 secondes, 50 secondes, 60 secondes, 90 secondes, 120 secondes, 150 secondes, 180 secondes, 240 secondes, 300 secondes, 360 secondes ou une combinaison de celles-ci ; ou dans lequel le PRP est obtenu en identifiant le changement de crête et de creux des signaux de pression oesophagienne pour chaque respiration et en le multipliant par la fréquence respiratoire.

4. - Procédé selon la revendication 1, dans lequel les conditions médicales relatives à un effort de respiration sont la probabilité d'un échec d'extubation.

5. - Support de stockage lisible par ordinateur non-transitoire stockant un ou plusieurs programmes pour évaluer des conditions médicales relatives à un effort de respiration chez un sujet qui en a besoin, le ou les programmes étant configurés pour être exécutés par un ou plusieurs processeurs d'un système informatique, le ou les programmes comprenant des instructions pour :

obtenir, à partir d'un premier capteur, des premiers signaux biométriques, les premiers signaux biométriques étant une pléthysmographie respiratoire par inductance (RIP), et obtenir, à partir d'un second capteur, des seconds signaux biométriques, les seconds signaux biométriques étant une pression oesophagienne (Pes) ; étalonner les signaux de RIP, les signaux de RIP étant étalonnés de façon à fournir un signal proportionnel à un volume courant ;

déterminer, à partir des premiers et seconds signaux biométriques, une valeur d'effort de respiration relative et tracer les signaux de RIP étalonnés contre les signaux de Pes pour obtenir une boucle de pression d'écoulement ;

afficher un rapport comprenant une interprétation de la valeur d'effort de respiration, l'interprétation étant mise à jour au fur et à mesure que des données supplémentaires relatives aux boucles de pression d'écoulement sont reçues.

Figure 1

100

110

User sets threshold
values for triggers
and artifact
management

102

Raw Input:
Esophageal
pressure,
spirometry and RIP
(Abd and RC)

104

Raw signals will be
stored if
measurements are
recorded

106

Low pass filter of
2 Hz applied to all
signals

112

Trigger procedures

108

Delay of 0.20s
applied to all
filtered signals

118

Input signals, Flow
Pressure loop,
Konno-Mead and
trends graphically
represented

114

Triggers and
filtered signals are
synced

115

Calibration of RIP
signals

116

Vt, RR,
WOB/PRP/PTP,
phase angles
calculated

120

Patient Ventilator
Asynchrony (PVA)
process

122

Weaning analysis
process

124

UAO severity
process

# Figure 2

# Figure 3

300

316

User sets Pes
trigger:
X decline in 0.10 s

302

Input:
Pes and
Pes (t – 0.10s)

304

Signal is averaged
over 0.1s
(artifact
management)

306

Is there 0.5*X
decline of Pes in
0.1s?

No

Yes

308

Is there a new
inspiration trigger
within Y ms?

Yes

310

'New' trigger is
ignored

No

312

Inspiration trigger

314

Has there been
an expiration
trigger?

Yes

No

## Figure 4A

Figure 4B

EP 3 148 426 B1

# Figure 5

500

508

User sets max RR
(X) for artifact
management

502

Input:
Esophageal
manometry (Pes)
and Pes trigger

504

Obtain Pes value at
inspiration trigger
(PEEP)

506

Determine
minimum Pes value
and multiply by RR
(PRP)

510

Is RR < X?

No →

512

PRP value is not
stored / displayed

Yes

514

Weaning          parameter
PRP < 250:              0
250   <  PRP  <  500:   1
500   <  PRP  <  1000:  2
1000 < PRP < 1500:      3
PRP   >  1500:          4

516

Weaning parameter
is scored per breath
and averaged over
60 seconds

518

< .50

.50 < x < 1.00

1.00 < x < 1.50

1.50 < x < 2.00

Weaning parameter
is translated into
'traffic light' output

2.00 < x < 2.50

2.50 < x < 3.00

3.00 < x < 3.50

> 3.50

518

Traffic light output
is displayed to user

## Figure 6

604

Determine
F(max)/Pes(min)
during CPAP
measurements
(K(F/P))

602

Input:
Calibrated RIP
Flow (F) and
Esophageal
manometry (Pes)

600

606

Transfer into F/Pes
derivative:
dF/dPes

608

Threshold value
(X):
K(F/P)*1.5*0.01

610

Is $\frac{dP}{dPes} < X$?

Yes

612

Each sample will be
given '1' as output

No

614

Each sample will be
given '0' as output

616

UAO Parameter:

$$\frac{sum\ of\ samples}{samples\ in\ breath}$$
*100%

618

Is UAO
Parameter
>15%?

Yes

620

Breath is annotated
as flow limited

No

622

Breath is annotated
as normal (severity
score = 0)

624

Weaning          parameter
PRP < 250:              0
250  <  PRP  <  500:   1
500  <  PRP  <  1000:  2
1000 < PRP < 1500:     3
PRP > 1500:            4

626

Severity scored per
breath is averaged
over 60 seconds

628

<.50

.50 < x < 1.00

1.00 < x < 1.50

1.50 < x < 2.00

Severity score is
translated into
'traffic light' output,
and displayed to
user

2.00 < x < 2.50

2.50 < x < 3.00

3.00 < x < 3.50

> 3.50

Figure 7

Figure 8

Pre Racemic Epinephrine

Post Racemic Epinephrine

UAO incidence and type vs Age
Distance Weighted Least Squares
UAO(L)   Subglottic(R)

Figure 9

EP 3 148 426 B1

Diaphragm vs Intercostal Paralysis

9 month old with infantile Botulism
Diaphragm paralysis: clockwise loop
RC expands, abdomen sucks in

2 year old with transverse myelitis
Intercostal paralysis: counter clockwise loop
RC sucks in, abdomen expands

Figure 10

Figure 11

Figure 12

Figure 13

## Figure 14

Figure 15

Pressure Rate Product Ratio between NIMV and HFNC as function of percent synchrony on NIMV

Figure 16

Figure 17

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62004034 B
- US 62162476 B

- WO 9841146 A1

**Non-patent literature cited in the description**

- **COATES AL ; DAVIS GM ; VALLINIS P ; OUTER-BRIDGE EW.** Liquid-filled esophageal catheter for measuring pleural pressure in preterm neonates. *J Appl Physiol,* 1989, vol. 67 (2), 889-893
- **BAGSHAW SM ; DELANEY A ; FARRELL C ; DRUMMOND J ; BRINDLEY PG.** Best evidence in critical care medicine. Steroids to prevent post-extubation airway obstruction in adult critically ill patients. *Can J Anaesth,* 2008, vol. 55 (6), 382-385
- **CHUNG Y-H ; CHAO T-Y ; CHIU C-T ; LIN M-C.** The cuff-leak test is a simple tool to verify severe laryngeal edema in patients undergoing long-term mechanical ventilation. *Crit Care Med,* 2006, vol. 34 (2), 409-414
- **CHENG KC ; HOU CC ; HUANG HC ; LIN SC ; ZHANG H.** Intravenous injection of methylprednisolone reduces the incidence of postextubation stridor in intensive care unit patients. *Critical Care Medicine,* May 2006, vol. 34 (5), 1345-50

- **KHEMANI R ; SCHNEIDER J ; MORZOV R ; MARKOVITZ B ; NEWTH CJ.** Pediatric Upper Airway Obstruction: Interobserver variability is the road to perdition. *J Crit Care,* 2013, vol. 28 (4), 490-497
- **ARAI YC ; FUKUNAGA K ; HIROTA S ; FUJIMOTO S ; ARAI Y-CP ; FUKUNAGA K ; HIROTA S ; FUJIMOTO S.** The effects of chin lift and jaw thrust while in the lateral position on stridor score in anesthetized children with adenotonsillar hypertrophy. *Anesth Analg,* vol. 99 (6), 1638-1641
- **KAPLAN V ; ZHANG JN ; RUSSI EW ; BLOCH KE.** Detection of inspiratory flow limitation during sleep by computer assisted respiratory inductive plethysmography. *Eur Respir J,* 2000, vol. 15 (3), 570-578